# EUROPEAN PATENT APPLICATION

(11) **EP 4 717 697 A1**
(43) Date of publication of application: **01.04.2026**
(21) Application number: 24203344.7
(22) Date of filing: 27.09.2024
(51) Int. Cl.: C07D 495/04, C07D 497/04, C09K 11/06

(54) **SELF-DOPED THIOPHENES FOR THERMALLY STABLE CAPACITOR APPLICATIONS**

(71) Applicant: Heraeus Epurio GmbH, 63450 Hanau (DE)
(72) Inventor: Hill, Stephen, 51368 Leverkusen (DE); Lövenich, Wilfried, 51368 Leverkusen (DE); Scheel, Arnulf, 51368 Leverkusen (DE); Merker, Udo, 51368 Leverkusen (DE)
(74) Representative: Herzog IP Patentanwalts GmbH

(57) **Abstract**

The present invention relates to a monomer of the general formula (I) 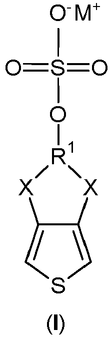 wherein
- **X** independently from each other represent O or S;
- **R¹** represents a linear or branched trivalent organic group;
- **M⁺** represents a cation, preferably a cation selected from the group consisting of H⁺, Li⁺, Na⁺, K⁺ and NH₄⁺, most preferably Na⁺.

The present invention also relates to an electrically conductive polymer comprising repeating units based on that monomer, to a liquid composition comprising that electrically conductive polymer, to a process for preparing a layered body, to a layered body, to an electronic device and to the use the monomer, the electrically conductive polymer and the liquid composition.

## Description

### FIELD OF THE INVENTION

The present invention relates to a monomer, to an electrically conductive polymer comprising repeating units based on that monomer, to a liquid composition comprising that electrically conductive polymer, to a process for preparing a layered body, to a layered body, to an electronic device and to the use the monomer, the electrically conductive polymer and the liquid composition.

### BACKGROUND OF THE INVENTION

A commercially available electrolyte capacitor as a rule is made of a porous metal electrode, an oxide layer serving as a dielectric on the metal surface, an electrically conductive material, usually a solid, which is introduced into the porous structure, an outer electrode (contacting), such as e.g., a silver layer, and further electrical contacts and an encapsulation. An electrolyte capacitor which is frequently used is the tantalum electrolytic capacitor, the anode electrode of which is made of the valve metal tantalum, on which a uniform, dielectric layer of tantalum pentoxide has been generated by anodic oxidation (also called *"formation"*)*.* A liquid or solid electrolyte forms the cathode of the capacitor. Aluminium capacitors in which the anode electrode is made of the valve metal aluminium, on which a uniform, electrically insulating aluminium oxide layer is generated as the dielectric by anodic oxidation, are furthermore frequently employed. Here also, a liquid electrolyte or a solid electrolyte forms the cathode of the capacitor. The aluminium capacitors are usually constructed as wound- or stacked-type capacitors.

π-conjugated polymers are particularly suitable as solid electrolytes in the capacitors described above because of their high electrical conductivity. π-conjugated polymers are also called conductive polymers or synthetic metals. They are increasingly gaining economic importance, since polymers have advantages over metals with respect to processability, weight and targeted adjustment of properties by chemical modification. Examples of known π-conjugated polymers are polypyrroles, polythiophenes, polyanilines, polyacetylenes, polyphenylenes and poly(p-phenylene-vinylenes), a particularly important polythiophene used industrially being poly(3,4-ethylenedioxythiophene) (PEDOT) since it has a very high conductivity in its oxidized form.

The solid electrolytes based on conductive polymers can be applied to the oxide layer in various ways. EP-A-0 340 512 describes, for example, the production of a solid electrolyte from 3,4-ethylenedioxythiophene and the use thereof in electrolytic capacitors. According to the teaching of this publication, 3,4-ethylenedioxythiophene is polymerized on to the oxide layer *in situ.* In addition to the *in situ* polymerization a process for the production of solid electrolytes in capacitors in which a dispersion comprising the already polymerized thiophene and a polyanion as a counterion, for example the PEDOT/PSS-dispersions (PEDOT = poly(3,4-ethylenedioxythiophene; PSS = polystyrene sulfonic acid) known from the prior art, is applied to the oxide layer and the dispersing agent is then removed by evaporation are also known from the prior art. Such a process for the production of solid electrolyte capacitors is disclosed, for example, in DE-A-10 2005 043 828.

However, PEDOT/PSS-dispersion are characterized by the disadvantage that they comprise a significant amount of PSS as a non-conducting inert material. Furthermore, due to the presence of PSS the size of the PEDOT/PSS-particles in the dispersions is sometimes too large to ensure that the particles also penetrate the smaller pores of the porous metal electrode. Finally, the maximum solids content of PEDOT/PSS-dispersions is often limited to values of about 3 wt.-%. In order to overcome these disadvantages, liquid compositions comprising derivatives of PEDOT have been prepared which are not characterized by the disadvantages of the known PEDOT/PSS-dispersions. Polythiophenes functionalized with sulfonate groups were developed initially. Due to the sulfonate groups, these polythiophenes are self-doped and do not require counter-ions such as PSS.

EP 1 122 274 A1 and US 9,781,905 B2, for example, disclose the preparation of functionalized π-conjugated polymers such as poly(4-(2,3-dihydrothieno[3,4-b][1,4]dioxin-2-ylmethoxy)-1-butanesulfonic acid) or poly(4-(2,3-dihydrothieno[3,4-b][1,4]dioxin-2-ylmethoxy)-2-butanesulfonic acid) (in the scientific literature both polymers are referred to as *"PEDOT-S"* or *"S-PEDOT"*) by oxidative polymerization of the corresponding monomer 4-(2,3-dihydrothieno[3,4-b][1,4]dioxin-2-ylmethoxy)-1-butanesulfonic acid and 4-(2,3-dihydrothieno[3,4-b][1,4]dioxin-2-ylmethoxy)-2-butanesulfonic acid (EDOT-S), respectively (in the scientific literature both monomers are referred to as *"EDOT-S"* or *"S-EDOT"*)*.* These monomers are usually prepared by sulfonation of thieno[3,4-b]-1,4-dioxin-2-methanol ("EDOT-MeOH") or mixtures of EDOT-MeOH and 3,4-(2-hydroxypropylendioxy)-thiophene ("ProDOT-MeOH") with 1,4-butanesultone (for the 1-butansulfonic acid derivative) or 2,4-butanesultone (for the 2-butansulfonic acid derivative), respectively.

However, the electrical conductivity of conductive layers prepared by the polymer solutions obtained by these approaches are still in need of improvement, particularly if these polymer solutions are used for the preparation of electrically conductive layers that are exposed to high temperatures. Conductive layers based on the known, self-doped conductive polymers carrying sulfonate groups often exhibit a significant increase in the surface resistance if exposed for prolonged times to high temperatures.

It was therefore an object of the present invention to overcome the disadvantages of the prior art in the field of self-doped conductive polymers.

Particularly, it was an object of the present invention to provide monomers based on which self-doped conductive polymers can be prepared that, compared to known self-doped conductive polymers carrying sulfonate groups, such as PEDOT-S, are characterized in that conductive layers prepared from these self-doped conductive polymers display an increased stability if exposed to high temperatures, whereby an increased stability characterizes the property of the conductive layer that its resistance increases as little as possible over time when being exposed to heat.

It was also an object of the present invention to provide self-doped conductive polymers that, compared to known self-doped conductive polymers carrying sulfonate groups, such as PEDOT-S, are characterized in that conductive layers prepared from these self-doped conductive polymers display an increased stability if exposed to heat.

Furthermore, it was an object of the present invention to provide liquid compositions comprising such self-doped conductive polymers by means of which electrically conductive layers can be prepared that, compared to conductive layers based on self-doped conductive polymers carrying sulfonic acid groups, such as PEDOT-S, display an increased stability if exposed to heat.

It was also an object of the present invention to provide electronic devices that comprise such conductive layers, particularly electrolytic capacitors the solid electrolyte layer of which is, or at least comprises, such a conductive layer, which, compared to electrolytic capacitors known from the prior art are characterized by an increased thermal stability. Here, an increased thermal stability characterizes the property of the capacitors that their electrical properties, particularly their electrical properties in terms of the equivalent series resistance (ESR) and/or the capacitance, changes as little as possible over time when being exposed to heat.

### SUMMARY OF THE INVENTION

A contribution to at least partly solving at least one, preferably more than one, of the above objects is made by the independent claims. The dependent claims provide preferred embodiments which contribute to at least partly solving at least one of the objects.

**|1a|** A contribution to solving at least one of the objects according to the invention is made by a 1^{st} embodiment of a monomer of the general formula (I) 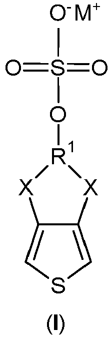 wherein
- **X** independently from each other represent O or S, wherein it is preferred that both **X-**atoms represent O;
- **R¹** represents a linear or branched trivalent organic group;
- **M⁺** represents a cation, preferably a cation selected from the group consisting of H⁺, Li⁺, Na⁺, K⁺ and NH₄⁺, most preferably Na⁺.

In contrast to the known thiophene-monomers carrying sulfonate groups or salts thereof that are used to prepare conductive polymers such as PEDOT-S, the monomers according to the invention are thiophene-monomers carrying sulfate groups (-O-SO₃H) or salts thereof (-O-SO₃M).

The term *"trivalent organic group"* as used herein to define group **R¹** encompasses any organic residue that can be linked with the two heteroatoms **X** that are bonded to the 3,4-position of the thiophene ring (preferably with the two oxygen atoms) and with one of the oxygen atoms of the sulfate group. Bonding to these groups can be accomplished *via* a carbon atom of the trivalent organic group **R¹** or *via* a heteroatom that may be present in that group, wherein bonding *via* a carbon atom is particularly preferred.

**|2a|** According to a preferred embodiment of the monomer according to the invention, the monomer has the general formula (I'): 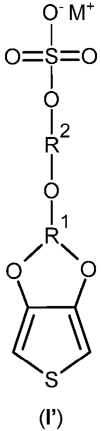 wherein
- **R¹** represents a linear or branched trivalent alkyl group, preferably a linear or branched trivalent alkyl group comprising 1 to 8 carbon atoms, preferably 2 to 6 carbon atoms, more preferably 3 to 5 carbon atoms and most preferably 3 carbon atoms;
- **R²** represents a linear or branched divalent alkyl group, preferably a linear or branched divalent alkyl group comprising 1 to 8 carbon atoms, preferably 1 to 6 carbon atoms, more preferably 2 to 4 carbon atoms and most preferably 2 carbon atoms or 3 carbon atoms;
- **M⁺** represents a cation, preferably a cation selected from the group consisting of H⁺, Li⁺, Na⁺, K⁺ and NH₄⁺, most preferably Na⁺.

This preferred embodiment is a 2^{nd} embodiment of the monomer according to the invention, that preferably depends on the 1^{st} embodiment.

**|3a|** According to a further preferred embodiment of the monomer according to the invention, the monomer has the general formula (**I'a**), the general formula (**I'b**) or the monomer is a monomer mixture comprising monomers of the general formula (**I'a**) and monomers of the general formula (**I'b**): 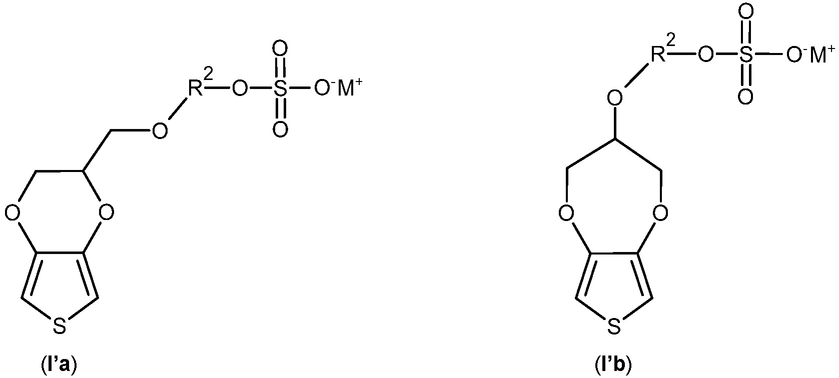 wherein
- **R²** represents a linear or branched divalent alkyl group, preferably a linear or branched divalent alkyl group comprising 1 to 8 carbon atoms, preferably 1 to 6 carbon atoms, more preferably 2 to 4 carbon atoms and most preferably 2 carbon atoms or 3 carbon atoms;
- **M⁺** represents a cation, preferably a cation selected from the group consisting of H⁺, Li⁺, Na⁺, K⁺ and NH₄⁺, most preferably Na⁺.

According to a particularly preferred embodiment of the monomer according to the present invention, **R²** is selected from the group consisting of -CH₂-CH₂-, -CH₂-CH₂-CH₂-, -CH(CH₃)-CH₂-, -CH₂-CH(CH₃)-, -CH₂-CH(CH₃)-CH₂-, -CH(CH₃)-CH₂-CH₂- and -CH₂-CH₂-CH(CH₃)-, wherein the first carbon atom (i.e., the left one) in each alkylene group indicates the bond to the oxygen atom of the sulfate group. This preferred embodiment is a 3^{rd} embodiment of the monomer according to the invention, that preferably depends on the 1^{st} or the 2^{nd} embodiment.

**|4a|** According to a further preferred embodiment of the monomer according to the invention, the monomer has the general formula (**I"a**), the general formula (**I"b**) or the monomer is a monomer mixture comprising monomers of the general formula (**I"a**) and monomers of the general formula (**I"b**): 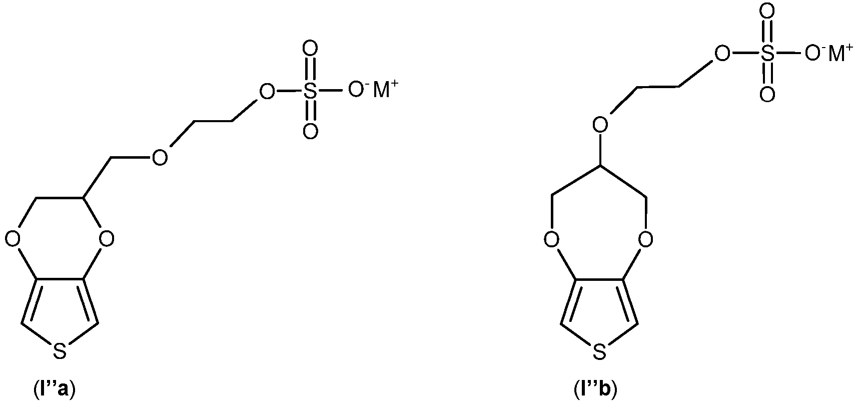 wherein M⁺ represents a cation, preferably a cation selected from the group consisting of H⁺, Li⁺, Na⁺, K⁺ and NH₄⁺, most preferably Na⁺. A monomer having the general formula (**I"a**) is also referred to as *"EDOT-OS"* (and in case of the sodium salt as "EDOT-OSNa"), whereas a monomer having the general formula (**I"b**) is also referred to as *"ProDOT-OS"* (and in case of the sodium salt as "ProDOT-OSNa"). This preferred embodiment is a 4^{th} embodiment of the monomer according to the invention, that preferably depends on any of the 1^{st} to the 3^{rd} embodiment.

|**5a**| According to a further preferred embodiment of the monomer according to the invention, the monomer, if being present in the form of a monomer mixture comprising monomers of the general formula (**I"a**) and monomers of the general formula (**I"b**), comprises monomers of the general formula (**I"a**) in an amount of at least 70 mol-%, preferably at least 80 mol-% and more preferably at least 90 mol-%, and monomers of the general formula (**I"b**) in an amount of not more than 30 mol-%, preferably not more than 20 mol-% and more preferably not more than 10 mol-%, in each case based on the total amount of monomers having the general formula (**I"a**) or (**I"b**) in the monomer mixture. This preferred embodiment is a 5^{th} embodiment of the monomer according to the invention, that preferably depends on the 4^{th} embodiment.

|**6a**| According to a further preferred embodiment of the monomer according to the invention, the monomer has the general formula (**I‴a**), the general formula (**I‴b**) or the monomer is a monomer mixture comprising monomers of the general formula (**I‴a**) and monomers of the general formula (**I‴b**): 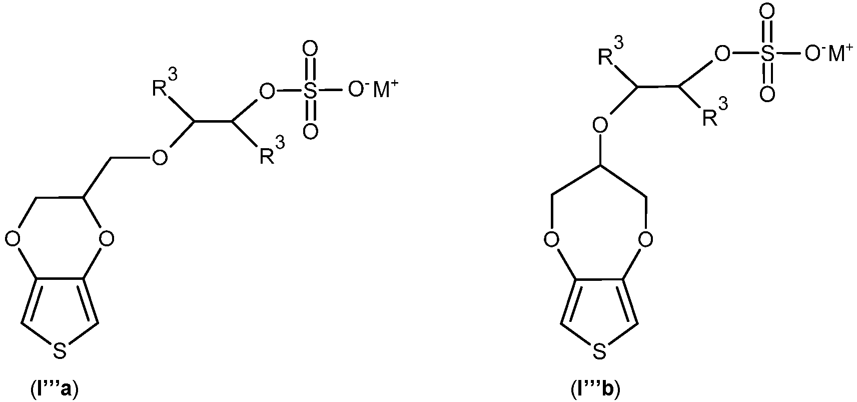 wherein
- one residue **R³** represents hydrogen and one residue **R³** represents a methyl group or an ethyl group, preferably a methyl group;
- **M⁺** represents a cation, preferably a cation selected from the group consisting of H⁺, Li⁺, Na⁺, K⁺ and NH₄⁺, most preferably Na⁺.

This preferred embodiment is a 6^{th} embodiment of the monomer according to the invention, that preferably depends on any of the 1^{st} to the 3^{rd} embodiment.

|**7a**| According to a further preferred embodiment of the monomer according to the invention, the monomer, if being present in the form of a monomer mixture comprising monomers of the general formula (**I"'a**) and monomers of the general formula (**I‴b**), comprises monomers of the general formula (**I"'a**) in an amount of at least 70 mol-%, preferably at least 80 mol-% and more preferably at least 90 mol-%, and monomers of the general formula (**I"'**b) in an amount of not more than 30 mol-%, preferably not more than 20 mol-% and more preferably not more than 10 mol-%, in each case based on the total amount of monomers having the general formula (**I"'a**) or (**I"'b**) in the monomer mixture. This preferred embodiment is a 7^{th} embodiment of the monomer according to the invention, that preferably depends on the 6^{th} embodiment.

|**8a**| According to a further preferred embodiment of the monomer according to the invention, the monomer comprises a compound of the general formula **R⁴**-O-SO₃M,
wherein **R⁴** represents a linear or branched alkenyl group, preferably a linear or branched alkenyl group comprising 1 to 8 carbon atoms, preferably 1 to 6 carbon atoms, more preferably 2 to 4 carbon atoms and most preferably 2 carbon atoms or 3 carbon atoms, and wherein **M⁺** represents a cation, preferably a cation selected from the group consisting of H⁺, Li⁺, Na⁺, K⁺ and NH₄⁺, most preferably Na⁺;
wherein the monomer comprises the monomers having formula (**I**) and the compound of the formula **R⁴**-O-SO₃M in a molar ratio "monomer of formula (**I**)": "compound of formula **R⁴**-O-SO₃M" of at least 9 : 1, preferably of at least 13 : 1 and more preferably of at least 24 : 1. Preferably, the number of carbon atoms in group **R⁴** corresponds to the number of carbon atoms ion group **R².** This preferred embodiment is an 8^{th} embodiment of the monomer according to the invention, that preferably depends on any of the 2^{nd} to the 7^{th} embodiment.

|**9a**| According to a further preferred embodiment of the monomer according to the invention, the monomer has the general formula (**I"a**), the general formula (**I"b**) or the monomer is a monomer mixture comprising monomers of the general formula (**I"a**) and monomers of the general formula (**I"b**): 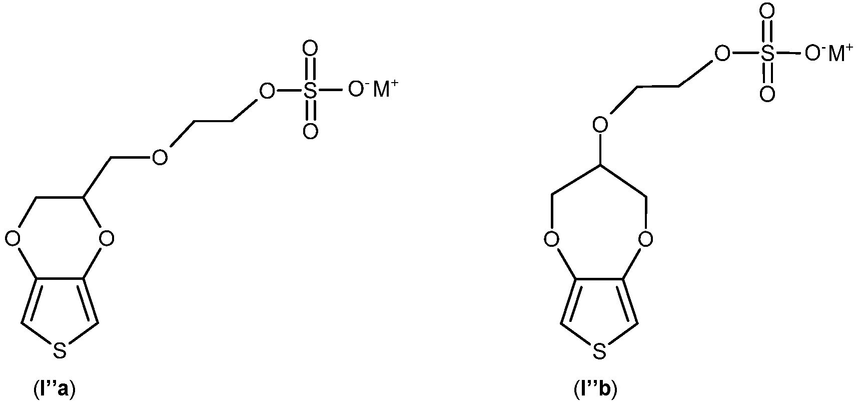
wherein **M⁺** represents a cation, preferably a cation selected from the group consisting of H⁺, Li⁺, Na⁺, K⁺ and NH₄⁺, most preferably Na⁺;
wherein the compound of formula **R⁴**-O-SO₃M has the general formula (**II**) 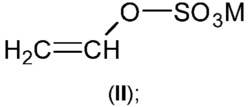
wherein **M⁺** represents a cation, preferably a cation selected from the group consisting of H⁺, Li⁺, Na⁺, K⁺ and NH₄⁺, most preferably Na⁺,
wherein the monomer or the mixture of monomers comprises the monomers having formula (**I"a**) or (**I"b**) and the compound of the general formula (**II**) in a molar ratio "monomer of formula (**I"a**) or (**I"b**)" : "compound of formula (**II**)" of at least 9 : 1, preferably of at least 13 : 1 and more preferably of at least 24 : 1. This preferred embodiment is a 9^{th} embodiment of the monomer according to the invention, that preferably depends on the 8^{th} embodiment.

|**1b**| A contribution to solving at least one of the objects according to the invention is also made by a 1^{st} embodiment of a process 1 for the preparation of an electrically conductive polymer, the process comprising the process steps:
α) dissolving or dispersing the monomer according to the invention, preferably the monomer according to any if its 1^{st} to the 11^{th} embodiment, in a solvent or dispersant, preferably in water, to obtain a solution or dispersion of the monomer;
β) oxidatively polymerizing the monomer in the solution or dispersion provided in process step α) to obtain a polymer composition in the form of a solution or dispersion comprising an electrically conductive polymer comprising repeating units of the general formula (**III**) 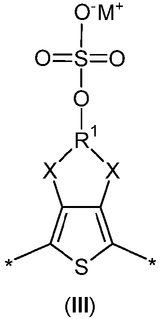 wherein symbol * indicates the bond to the neighboured repeating units and wherein
   - **X** independently from each other represent O or S, wherein it is preferred that both **X**-atoms represent O;
   - **R¹** represents a linear or branched trivalent organic group;
   - **M⁺** represents a cation, preferably a cation selected from the group consisting of H⁺, Li⁺, Na⁺, K⁺ and NH₄⁺, most preferably Na⁺;
γ) optionally purification of the solution or dispersion comprising the electrically conductive polymer obtained in process step β), preferably by means of ion filtration;
δ) optionally diluting the solution or dispersion comprising the electrically conductive polymer obtained in process step β) or in process step γ), preferably diluting the solution or dispersion with water.

The electrically conductive polymers thus obtained are self-doped polythiophenes carrying sulfate groups. For the purposes of the present invention, *"self-doped conductive polymer"* means a conductive polymer which has positive charges along the polymer chain (not shown formula (**III**) above) and in which these positive charges are at least partially compensated for by the negatively charged counterions (*i.e*., the sulfate groups) which are covalently bonded to the conductive polymer.

|**2b**| In a preferred embodiment of process 1 according to the invention, the solution or dispersion provided in process step α) comprises the functionalized thiophene monomer having the general formula (**I**) in an amount in the range from 1 to 40 wt.-%, preferably in the range from 2 to 30 wt.-% and more preferably in the range from 3 to 20 wt.-%, in each case based on the total weight of the solution or dispersion provided in process step α). This preferred embodiment is a 2^{nd} embodiment of process 1 according to the invention, that preferably depends on the 1^{st} embodiment.

|**3b**| In a further preferred embodiment of process 1 according to the invention, the solution or dispersion provided in process step α) further comprises at least one oxidizing agent. This preferred embodiment is a 3^{rd} embodiment of process 1 according to the invention, that preferably depends on the 1^{st} or the 2^{nd} embodiment.

|**4b**| In a further preferred embodiment of process 1 according to the invention, the solution or dispersion provided in process step α) is an aqueous solution or dispersion and the pH of the aqueous solution or dispersion provided in process step α) is adjusted to a value of less than 2.5, preferably less than 2.0 and more preferably less than 1.5 using an organic or inorganic acid, preferably an acid selected from the group consisting of formic acid, acetic acid, lactic acid, propionic acid, citric acid, malic acid, fumaric acid, sulfuric acid, sulfonic acid, nitric acid, phosphonic acid, phosphoric acid or a mixture of at least two of these acids, wherein the use of sulfuric acid is particularly preferred. This preferred embodiment is a 4^{th} embodiment of process 1 according to the invention, that preferably depends on any of the 1^{st} to the 3^{rd} embodiment.

|**5b**| In a further preferred embodiment of process 1 according to the invention, the oxidative polymerization in process step β) is performed under an inert gas atmosphere of nitrogen, argon, carbon dioxide or a mixture thereof. This preferred embodiment is a 5^{th} embodiment of process 1 according to the invention, that preferably depends on any of the 1^{st} to the 4^{th} embodiment.

|**6b**| In a further preferred embodiment of process 1 according to the invention, the oxidative polymerization in process step β) is performed under a pressure that is equal to or above the vapor pressure of the aqueous solution or dispersion during the polymerization reaction in process step β). This preferred embodiment is a 6^{th} embodiment of process 1 according to the invention, that preferably depends on any of the 1^{st} to the 5^{th} embodiment.

|**7b**| In a further preferred embodiment of process 1 according to the invention, the oxidative polymerization in process step β) is performed under a reduced pressure of not more than 0.8 bar. This preferred embodiment is a 7^{th} embodiment of process 1 according to the invention, that preferably depends on any of the 1^{st} to the 6^{th} embodiment.

|**8b**| In a further preferred embodiment of process 1 according to the invention, the purification in process step γ) is accomplished by means of ultrafiltration and/or by means of a treatment with ion exchanger. This preferred embodiment is an 8^{th} embodiment of process 1 according to the invention, that preferably depends on any of the 1^{st} to the 7^{th} embodiment.

|**9b**| In a further preferred embodiment of process 1 according to the invention, the dilution in process step δ) is done to such an extent that the solids content of the polymer composition is in the range from 0.1 to 25 wt.-%, preferably in the range from 0.25 to 10 wt.-% and more preferably in the range from 0.5 to 5 wt.-%, in each case based on the total weight of the polymer composition. This preferred embodiment is an 9^{th} embodiment of process 1 according to the invention, that preferably depends on any of the 1^{st} to the 8^{th} embodiment.

|**1c**| A contribution to solving at least one of the objects according to the invention is also made by a 1^{st} embodiment of an electrically conductive polymer comprising repeating units of the general formula (III) 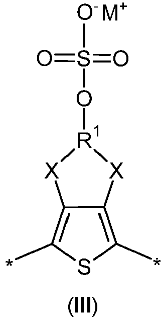 wherein symbol * indicates the bond to the neighboured repeating units and wherein
- **X** independently from each other represent O or S, wherein it is preferred that both **X-**atoms represent O;
- **R¹** represents a linear or branched trivalent organic group;
- **M⁺** represents a cation, preferably a cation selected from the group consisting of H⁺, Li⁺, Na⁺, K⁺ and NH₄⁺, most preferably Na⁺.

|**2c**| According to a preferred embodiment of the electrically conductive polymer according to the invention, the electrically conductive polymer comprises the repeating units of the general formula (**III**) in an amount of at least 75 mol-%, preferably of at least 85 mol-%, more preferably of at least 95 mol-% and most preferably of at least 99 mol-%, in each case based on the total amount of repeating units in the electrically conductive polymer. In this context it is particularly preferred that the electrically conductive polymer is a homopolymer that consists of repeating units of the general formula (III). This preferred embodiment is a 2^{nd} embodiment of the electrically conductive polymer monomer according to the invention, that preferably depends on the 1^{st} embodiment.

|**3c**| According to a further preferred embodiment of the electrically conductive polymer according to the invention, the electrically conductive polymer comprises repeating units of the general formula (**III**') 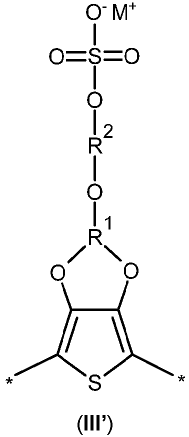 wherein symbol * indicates the bond to the neighboured repeating units and wherein
- **R¹** represents a linear or branched trivalent alkyl group, preferably a linear or branched trivalent alkyl group comprising 1 to 8 carbon atoms, preferably 2 to 6 carbon atoms, more preferably 3 to 5 carbon atoms and most preferably 3 carbon atoms;
- **R²** represents a linear or branched divalent alkyl group, preferably a linear or branched divalent alkyl group comprising 1 to 8 carbon atoms, preferably 1 to 6 carbon atoms, more preferably 2 to 4 carbon atoms and most preferably 2 carbon atoms or 3 carbon atoms;
- **M⁺** represents a cation, preferably a cation selected from the group consisting of H⁺, Li⁺, Na⁺, K⁺ and NH₄⁺, most preferably Na⁺.

According to a particularly preferred embodiment of the electrically conductive polymer according to the present invention, **R²** is selected from the group consisting of -CH₂-CH₂-, -CH₂-CH₂-CH₂-, -CH(CH₃)-CH₂-, -CH₂-CH(CH₃)-, -CH₂-CH(CH₃)-CH₂-, -CH(CH₃)-CH₂-CH₂- and -CH₂-CH₂-CH(CH₃)-, wherein the first carbon atom (i.e., the left one) in each alkylene group indicates the bond to the oxygen atom of the sulfate group. This preferred embodiment is a 3^{rd} embodiment of the electrically conductive polymer according to the invention, that preferably depends on the 1^{st} embodiment.

|**4c**| According to a further preferred embodiment of the electrically conductive polymer according to the invention, the electrically conductive polymer comprises repeating units of the general formula (**III'a**), repeating units of the general formula (**III'b**) or a combination of repeating units of the general formula (**III'a**) and of the general formula (**III'b**): 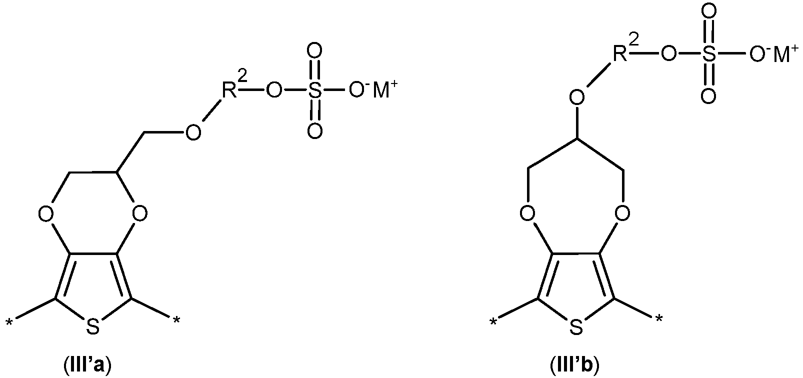 wherein* indicates the bond to the neighboured repeating units and wherein
- **R²** represents a linear or branched divalent alkyl group, preferably a linear or branched divalent alkyl group comprising 1 to 8 carbon atoms, preferably 1 to 6 carbon atoms, more preferably 2 to 4 carbon atoms and most preferably 2 carbon atoms or 3 carbon atoms;
- **M⁺** represents a cation, preferably a cation selected from the group consisting of H⁺, Li⁺, Na⁺, K⁺ and NH₄⁺, most preferably Na⁺.

This preferred embodiment is a 4^{th} embodiment of the electrically conductive polymer according to the invention, that preferably depends on any of the 1^{st} to the 3^{rd} embodiment.

|**5c**| According to a further preferred embodiment of the electrically conductive polymer according to the invention, the electrically conductive polymer comprises, preferably consists of, repeating units of the general formula (**III"a**), repeating units of the general formula (**III"b**) or a combination of repeating units of the general formula (**III"a**) and of the general formula (**III"b**): 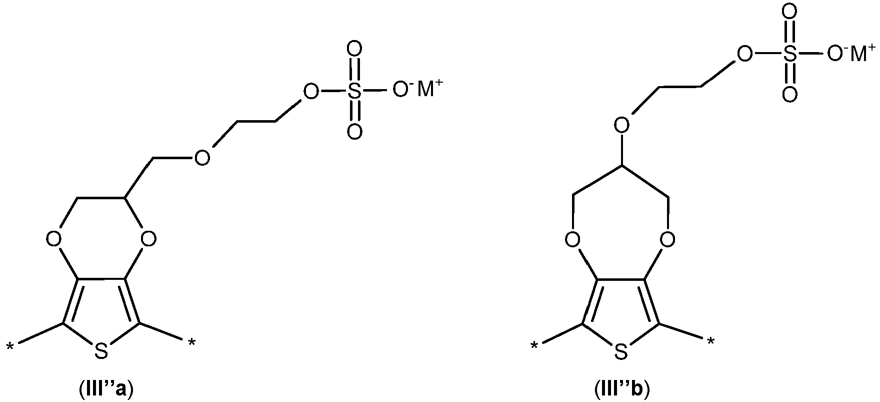 wherein* indicates the bond to the neighboured repeating units and wherein **M⁺** represents a cation, preferably a cation selected from the group consisting of H⁺, Li⁺, Na⁺, K⁺ and NH₄⁺, most preferably Na⁺. This preferred embodiment is a 5^{th} embodiment of the electrically conductive polymer according to the invention, that preferably depends on the 4^{th} embodiment.

|**6c**| According to a further preferred embodiment of the electrically conductive polymer according to the invention, the electrically conductive polymer comprises repeating units of the general formula (**III"a**) in an amount of at least 70 mol-%, preferably at least 80 mol-% and more preferably at least 90 mol-%, and repeating units of the general formula **(III"b)** in an amount of not more than 30 mol-%, preferably not more than 20 mol-% and more preferably not more than 10 mol-%, in each case based on the total amount of repeating units of the general formula (**III"a**) or (**III"b**) in the electrically conductive polymer. This preferred embodiment is a 6^{th} embodiment of the electrically conductive polymer according to the invention, that preferably depends on the 5^{th} embodiment.

|**7c**| According to a further preferred embodiment of the electrically conductive polymer according to the invention, the electrically conductive polymer comprises, preferably consists of, repeating units of the general formula (III‴a), repeating units of the general formula (**III‴b**) or a combination of repeating units of the general formula (**III‴a**) and of the general formula (**III‴b**): 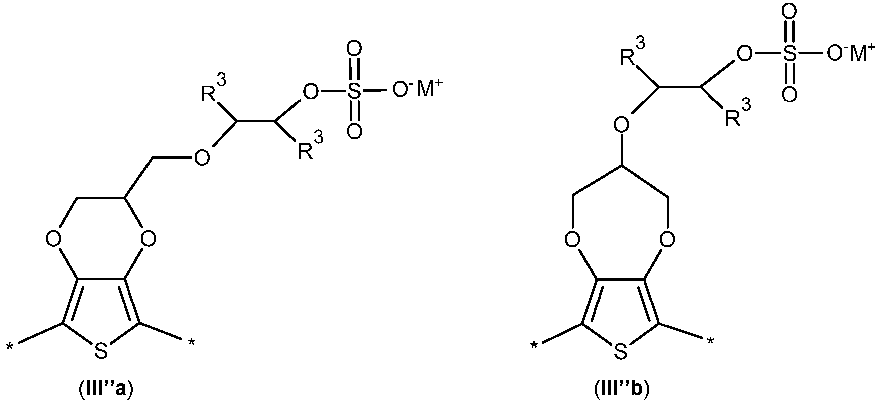 wherein* indicates the bond to the neighboured repeating units and wherein
- one residue **R³** represents hydrogen and one residue **R³** represents a methyl group or an ethyl group, preferably a methyl goup;
- M⁺ represents a cation, preferably a cation selected from the group consisting of H⁺, Li⁺, Na⁺, K⁺ and NH₄⁺, most preferably Na⁺.

This preferred embodiment is a 7^{th} embodiment of the electrically conductive polymer according to the invention, that preferably depends on the any of the 1^{st} to the 4^{th} embodiment.

|**8c**| According to a further preferred embodiment of the electrically conductive polymer according to the invention, the electrically conductive polymer comprises repeating units of the general formula (**III‴a**) in an amount of at least 70 mol-%, preferably at least 80 mol-% and more preferably at least 90 mol-%, and repeating units of the general formula (**III‴b**) in an amount of not more than 30 mol-%, preferably not more than 20 mol-% and more preferably not more than 10 mol-%, in each case based on the total amount of repeating units of the general formula (**III‴a**) or (**III‴b**) in the electrically conductive polymer. This preferred embodiment is an 8^{th} embodiment of the electrically conductive polymer according to the invention, that preferably depends on the 7^{th} embodiment.

|**1d**| A contribution to solving at least one of the objects according to the invention is also made by a 1^{st} embodiment of a liquid composition comprising
**i)** a dispersant (also referred to as a "dispersing agent");
**ii)** the electrically conductive polymer according to the invention, preferably the electrically conductive polymer according to any of its 1^{st} to the 8^{th} embodiment.

The liquid composition may be a dispersion or solution. The term *"dispersion"* as used in context with the present invention general refers to any liquid composition in which a self-doped polythiophene according to the present invention is somehow distributed in a homogeneous phase that is formed by the liquid dispersant **i)** (dispersant **i)** thus forms the liquid phase of the dispersion). It should be noted that transitions between a *"dispersion"* and a *"solution"* can be fluid. For that reason, no distinction is made hereafter between the terms *"dispersed"* and *"dissolved".* Likewise, no distinction is made between *"dispersion"* and *"solution"* or between *"dispersant"* and *"solvent'.* Rather these terms are used synonymously hereafter.

|**2d**| According to a preferred embodiment of the liquid composition according to the invention, the dispersant **i)** (or solvent) is selected from the group consisting of water, aliphatic alcohols, such as methanol, ethanol, isopropanol and butanol, diacetone alcohols, ethylene glycol and glycerol, aliphatic ketones, such as acetone and methyl ethyl ketone, aliphatic nitrites, such as acetonitrile, glycol ethers, such polyethylene glycol methyl ether, and a mixture of at least two of these dispersants, wherein water is most preferred as the dispersant **i).** This preferred embodiment is a 2^{nd} embodiment of the liquid composition according to the invention, that preferably depends on the 1^{st} embodiment.

|**3d**| According to a further preferred embodiment of the liquid composition according to the invention, the liquid composition comprises the electrically conductive polymer **ii)** in an amount in the range from 0.1 to 25 wt.-%, preferably in the range from 0.25 to 10 wt.-% and more preferably in the range from 0.5 to 5 wt.-%, in each case based on the total weight of the liquid composition. This preferred embodiment is a 3^{rd} embodiment of the liquid composition according to the invention, that preferably depends on the 1^{st} or the 2^{nd} embodiment.

|**4d**| According to a further preferred embodiment of the liquid composition according to the invention, the liquid composition has a viscosity (measured with a rheometer at 20 °C and a shear rate of 100 s⁻¹) in the range from 0.01 and 1,000 mPa×s, preferably in the range from 1 to 500 mPa×s, more preferably in the range from 1 to 250 mPa×s and most preferably in the range from 5 to 25 mPa×s. This preferred embodiment is a 4^{th} embodiment of the liquid composition according to the invention, that preferably depends on any of the 1^{st} to the 3^{rd} embodiment.

|**5d**| According to a further preferred embodiment of the liquid composition according to the invention, a conductive layer prepared with the liquid composition has a conductivity of at least 10 S/cm, preferably of at least 50 S/cm, more preferably of at least 100 S/cm, more preferably of at least 200 S/cm and even more preferably of at least 300 S/cm. This preferred embodiment is a 5^{th} embodiment of the liquid composition according to the invention, that preferably depends on any of the 1^{st} to the 4^{th} embodiment.

|**6d**| According to a further preferred embodiment of the liquid composition according to the invention, the liquid composition comprises further additives, preferably further additives selected from the group consisting of surface-active substances, adhesion promoters, additives which increase the conductivity, organic binders or mixtures of at least two of these further additives. This preferred embodiment is a 6^{th} embodiment of the liquid composition according to the invention, that preferably depends on any of the 1^{st} to the 5^{th} embodiment.

|**1e**| A contribution to solving at least one of the objects according to the invention is also made by a 1^{st} embodiment of a process 2 for preparing a layered body, comprising the process steps:
**A)** providing a substrate;
**B)** applying the liquid composition according to the invention, preferably a liquid composition according to any of its 1^{st} to the 6^{th} embodiment, onto a surface of the substrate;
**C)** at least partially removing the dispersant **i)** for the formation of a conductive layer that at least partially covers a surface of the substrate.

|**2e**| According to a preferred embodiment of process 2 according to the invention, the layered body is part of an electrolyte capacitor, wherein the substrate is a porous electrode body made of an electrode material, wherein a dielectric layer at least partially covers a surface of this electrode material and wherein the electrically conductive layer is a solid electrolyte layer or part of a solid electrolyte layer that at least partially covers a surface of the dielectric layer. This preferred embodiment is a 2^{nd} embodiment of process 2 according to the invention, that preferably depends on the 1^{st} embodiment.

|**3e**| According to a further preferred embodiment of process 2 according to the invention, the process comprises the steps of:
**A)** provision of a porous electrode body made of an electrode material, wherein a dielectric layer at least partially covers a surface of this electrode material;
**B)** introduction of the liquid composition according to the invention, preferably of a liquid composition according to any of its 1^{st} to the 6^{th} embodiment, into at least a part of the porous electrode body provided in process step **A)** so as to apply the dispersion onto at least a part of the surface of the porous electrode body, preferably onto at least a part of the surface of the dielectric layer;
**C)** at least partial removal of the dispersant **i)** for the formation of a solid electrolyte that at least partially covers a surface of the dielectric layer;
**D)** optionally application of a dispersion containing a conductive polymer, preferably a dispersion comprising a polythiophene and a dispersant, more preferably a dispersion comprising a dispersant and particles of a complex of a polythiophene and a polymeric anion, even more preferably a PEDOT/PSS-dispersion, onto at least a part of the surface of the solid electrolyte layer and at least partial removal of the dispersant for the formation of a polymeric outer layer that at least partially covers a surface of the solid electrolyte layer.

This preferred embodiment is a 3^{rd} embodiment of process 2 according to the invention, that preferably depends on the 1^{st} or the 2^{nd} embodiment.

|**4e**| According to a further preferred embodiment of process 2 according to the invention, the layered body is an aluminum capacitor or a tantalum capacitor. This preferred embodiment is a 4^{th} embodiment of process 2 according to the invention, that preferably depends to any of its 1^{st} to the 3^{rd} embodiment.

|**1f**| A contribution to solving at least one of the objects according to the invention is also made by a layered body, comprising
**I)** a substrate;
**II)** a conductive layer that at least partially covers a surface of the substrate;
wherein the conductive layer comprises the electrically conductive polymer according to the invention, preferably the electrically conductive polymer according to any of its 1^{st} to the 8^{th} embodiment.

|**2f**| According to a preferred embodiment of the layered body according to the invention, the conductive layer has a conductivity of at least 10 S/cm, preferably of at least 50 S/cm, more preferably of at least 100 S/cm, more preferably of at least 200 S/cm and even more preferably of at least 300 S/cm. This preferred embodiment is a 2^{nd} embodiment of the layered body according to the invention, that preferably depends on the 1^{st} embodiment.

|**3f**| According to a further preferred embodiment of the layered body according to the invention, the layered body is part of an electrolyte capacitor, wherein the substrate is a porous electrode body made of an electrode material, wherein a dielectric layer at least partially covers a surface of this electrode material and wherein the electrically conductive layer is a solid electrolyte layer or part of a solid electrolyte layer that at least partially covers a surface of the dielectric layer. This preferred embodiment is a 3^{rd} embodiment of the layered body according to the invention, that preferably depends on the 1^{st} or the 2^{nd} embodiment.

|**1g**| A contribution to solving at least one of the objects according to the invention is also made by an electronic device, comprising the layered body according to the invention, preferably the layered body according to any its 1^{st} to the 3^{rd} embodiment.

|**2g**| According to a preferred embodiment of the electronic device according to the invention, the electronic device is an electrolytic capacitor, wherein the conductive layer is a solid electrolyte layer or part of a solid electrolyte layer. This preferred embodiment is a 2^{nd} embodiment of the electronic device according to the invention, that preferably depends on the 1^{st} embodiment.

|**3g**| According to a further preferred embodiment of the electronic device according to the invention, the electronic device is an aluminum capacitor or a tantalum capacitor. This preferred embodiment is a 3^{rd} embodiment of the electronic device according to the invention, that preferably depends on the 1^{st} or the 2^{nd} embodiment.

|**1h**| A contribution to solving at least one of the objects according to the invention is also made by the use of the monomer according to the invention, preferably the monomer according to any of its 1^{st} to the 11^{th} embodiment, of the electrically conductive polymer according to the invention, preferably of the electrically conductive polymer according to any of its 1^{st} to the 8^{th} embodiment, or of the liquid composition according to the invention, preferably of the liquid composition according to any of its 1^{st} to the 6^{th} embodiment, for the formation of an electrically conductive layer.

|**2h**| According to preferred embodiment of the use according to the invention, the electrically conductive layer is a solid electrolyte layer or is part of a solid electrolyte layer in an electrolytic capacitor. This preferred embodiment is 2^{nd} embodiment of the use according to the invention, that preferably depends on the 1^{st} embodiment.

|**3h**| According to further preferred embodiment of the use according to the invention, the electrolytic capacitor is an aluminum capacitor or a tantalum capacitor. This preferred embodiment is 3^{rd} embodiment of the use according to the invention, that preferably depends on the 2^{nd} embodiment.

### Process for the preparation of thiophene monomers carrying sulfate groups

The monomers according to the present invention carrying sulfate groups that have the general formula (I) can be obtained by reacting monomer precursors carrying functional groups such as those having the formula (IV) 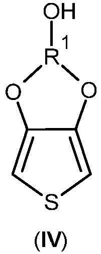 wherein R¹ is as defined above, with organic sulfates such as those having the formula (V) 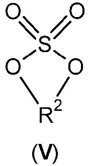 in which R² is as defined above in the presence of bases, such as sodium hydride.

In case of EDOT-OS and ProDOT-OS, the precursors EDOT-OH or ProDOT-OH (or a mixture of these precursors) 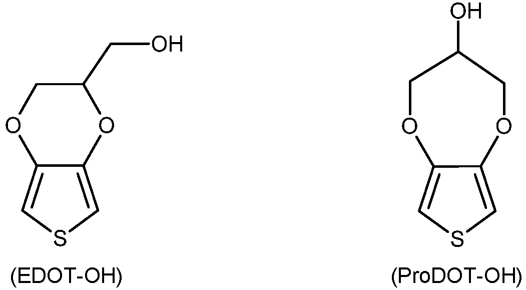 may be reacted with ethylene sulfate 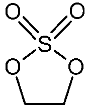 to obtain monomers of the general formula (**I"a**) or (**I"b**) as shown in the experimental section of the present application. In the alternative, EDOT-OH and ProDOT-OH may also be reacted with 1,2-propylene sulfate 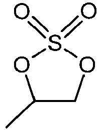 to obtain monomers of the general formula (**I"'a**) or (**I‴b**). Also, other cyclic sulfates such as 1,3-propylene sulfate or 1,3-butylene sulfate, 1,3,2-Dioxathiane-4,6-diethyl-2,2-dioxide, 1,3,2-Dioxathiane-4,6-dimethyl-2,2-dioxide, 1,3,2-Dioxathiane-4,4,6-trimethyl-2,2-dioxide, 5,5-dimethyl-1,3,2-dioxathiane 2,2-dioxide can likewise be used.

The reaction can be performed in water or in organic solvents. Preferred organic solvents are ethers selected from the group consisting of diethyl ether, methyl tertiary butyl ether, ethyl tertiary butyl ether, di-tertiary butyl ether, diisopropyl ether, dimethoxymethane, tetrahydrofuran, 2-methyltetrahydrofuran and tetrahydropyran or combinations thereof, esters selected from the group consisting of methyl acetate, ethyl acetate, isopropyl acetate, n-propyl acetate, isobutyl acetate, and n-butyl acetate, or combinations thereof, ketones selected from the group consisting of: methyl isobutyl ketone and methyl isopropyl ketone, toluene or a combination thereof. In case of EDOT-OH and ProDOT-OH as educts, tetrahydrofuran is a particularly preferred solvent.

In case of monomers according to the present invention that have been prepared using alkylene sulfates as a reactant, monoalkenyl ester sulfates of the general formula **R⁴**-O-SO₃M can be formed, wherein **R⁴** represents a linear or branched alkenyl group, preferably a linear or branched alkenyl group comprising 1 to 8 carbon atoms, preferably 1 to 6 carbon atoms, more preferably 2 to 4 carbon atoms and most preferably 2 carbon atoms or 3 carbon atoms, and wherein M⁺ represents a cation, preferably a cation selected from the group consisting of H⁺, Li⁺, Na⁺, K⁺ and NH₄⁺, most preferably Na⁺. In case of EDOT-OS and ProDOT-OS, this by-product is Ethenyl-OS or a salt thereof having the general formula (**II**)

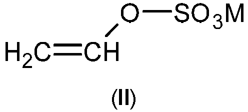

Here, it has been shown to be particularly advantageous if in the final product the content of these monoalkenyl ester sulfates is further reduced. This reduction, for example, can be accomplished by using common purification methods such as HPLC, purification by means of crystallization, precipitation, silica gel chromatography *etc..*

### Process for the preparation of polythiophenes carrying sulfate groups

The electrically conductive polymers of the general formula (III) that are based on the above-described thiophene monomers carrying sulfate groups can be obtained by means of process 1 according to the invention, this process comprising the process steps:
α) dissolving or dispersing the monomer according to the present invention, preferably the monomer according to any of its 1^{st} to the 7^{th} embodiment, in a solvent or dispersant, preferably in water, to obtain a solution or dispersion of the monomer;
β) oxidatively polymerizing the monomer in the solution or dispersion provided in process step α) to obtain a polymer composition in the form of a solution or dispersion comprising an electrically conductive polymer comprising repeating units of the general formula (**III**) 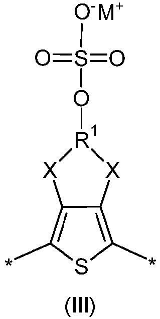 wherein symbol * indicates the bond to the neighboured repeating units and wherein
   - **X** independently from each other represent O or S, wherein it is preferred that both **X**-atoms represent O;
   - **R¹** represents a linear or branched trivalent organic group;
   - **M⁺** represents a cation, preferably a cation selected from the group consisting of H⁺, Li⁺, Na⁺, K⁺ and NH₄⁺, most preferably Na⁺;
γ) optionally purification of the solution or dispersion comprising the electrically conductive polymer obtained in process step β), preferably by means of ion filtration;
δ) optionally diluting the solution or dispersion comprising the electrically conductive polymer obtained in process step β) or in process step γ), preferably diluting the solution or dispersion with water.

The oxidation reaction that is performed in process step β) can be catalyzed by a chemical oxidizing agent, by electrochemical oxidation or by a combination of both methods. In case of an electrochemical oxidation an electrode functions as the oxidizing agent.

Suitable oxidizing agents used as chemical oxidizing agents are salts of heavy metals, preferably iron salts, more preferably FeCl₃, FeSO₄ and iron(lll) salts of aromatic and aliphatic sulfonic acids, H₂O₂, K₂Cr₂O₇, salts of a salt of a peroxodisulfate, such as K₂S₂O₈, Na₂S₂O₈, KMnO₄, alkali metal perborates, and alkali metal or ammonium persulfates, or mixtures of these oxidants. Particularly preferred are salts of a heavy metal, salts of a peroxodisulfate or a mixture thereof. Further suitable oxidants are described, for example, in Handbook of Conducting Polymers (Ed. Skotheim, T. A.), Marcel Dekker: New York, 1986, Vol. 1, pages 46-57. Particularly preferred oxidizing agents b) are salts of a peroxodisulfate, in particular K₂S₂O₈, Na₂S₂O₈, iron salts, in particular iron(III) chloride, or mixtures of salts of a peroxodisulfate and at least one further compound that catalyzes the cleavage of the peroxodisulfate, like mixtures of salts of a peroxodisulfate and iron salts. According to a particularly preferred embodiment of process 1 according to the invention, the oxidizing agent is a mixture of FeSO₄ and Na₂S₂O₈.

Suitable solvents or dispersants that can be used in the polymerization process are water, water-miscible solvents, in particular those selected from the group consisting aliphatic alcohols, such as methanol, ethanol, isopropanol and butanol, diacetone alcohols, ethylene glycol and glycerol, aliphatic ketones, such as acetone and methyl ethyl ketone, aliphatic nitrites, such as acetonitrile, glycol ethers, such polyethylene glycol methyl ether, or a mixture of at least two of these solvents, in particular a mixture of water and a water-miscible solvent. The most preferred solvent or dispersant, however, is water. In case of EDOT-OS or ProDOT-OS as the thiophene monomers carrying sulfate groups, process 1 according to the invention therefore enables the production of an aqueous solution or dispersion comprising poly(EDOT-OS)-homopolymers, poly(ProDOT-OS)-homopolymers or poly(EDOT-OS/ProDOT-OS)-copolymers.

The concentration of the monomer of the general formula (**I**) in the solution or dispersion provided in process step α) is preferably in a range from 1 to 40 wt.-%, preferably in the range from 2 to 30 wt.-% and more preferably in the range from 3 to 20 wt.-%, in each case based on the total weight of the solution or dispersion.

There are different ways of preparing the solution or dispersion provided in process step α). The monomer of the general formula (**I**) can be dissolved or dispersed in the solvent or dispersant, followed by the addition of the oxidizing agent(s) (which can also be dissolved or dispersed in a solvent or dispersant separately), or the oxidizing agent(s) is/are first dissolved or dispersed in the solvent or dispersant, followed by the addition of the monomer of the general formula (**I**) (which can also be dissolved or dispersed in a solvent or dispersant separately). If more than one oxidizing agent is used, like a mixture of FeSO₄ and Na₂S₂O₈, it is furthermore possible to first mix one of these components with the monomer of the general formula (**I**) and the solvent or dispersant and finally add the second oxidizing agent.

The polymerization reaction in process step β) is preferably performed at a temperature in the range from -20°C to 150°C, preferably from 0°C to 75°C more preferably from 0 to 15°C and for a duration of preferably 1 to 48 hours, more preferably for 5 to 20 hours.

After the polymerization reaction is completed, the solution or dispersion comprising the polymer of the general formula (**III**), preferably the aqueous solution comprising the polymer of the general formula (**III**), may be further purified, for example by means of filtration, in particular by means of ultrafiltration, and/or by a treatment with ion exchanger in a further process step γ), in particular by a treatment with an anion exchanger and a cation exchanger, for the purpose of further purification. It is also possible to add further additives as described below in connection with the process 1 according to the present invention.

Moreover, the solution or dispersion comprising the polymer of the general formula (**III**), preferably the aqueous solution comprising the polymer of the general formula (**III**), that is obtained in process step β) and/or that is obtained after purification in process step γ), may further be diluted, preferably using any of the solvents or dispersants mentioned above in connection with process step β), more preferably using water, in a further process step δ). By means of such a dilution step the solids content of the solution or dispersion is preferably adjusted to be in the range from 0.1 to 25 wt.-%, preferably in the range from 0.25 to 10 wt.-% and more preferably in the range from 0.5 to 5 wt.-%, in each case based on the total weight of the solution or dispersion.

Furthermore, as the polymer of the general formula (**III**) obtained after polymerization in process step β) is usually present in the form of particles, the particle size distribution of the polymer in the solution or dispersion obtained in process step β) or that - after further purification and or after dilution - is obtained in process step γ) or process step δ), can be adjusted by a treatment of the solution or dispersion with ultrasound, wherein the energy input is preferably between 10 - 1000 Watts/liter (W/I), more preferably between 20-500 W/I and the ultrasound frequency is preferably between 20 - 200 kHz, by a treatment of the solution or dispersion with high pressure homogenization, wherein pressures higher than 100 bar, preferably higher than 500 bar and most preferably higher than 1500 bar are applied preferably multiple times, or by a treatment of the solution or dispersion with heat, wherein the heat treatment preferably comprises a treatment of the solution or dispersion at a temperature in the range from 40 to 100°C, preferably in the range from 50 to 95°C for a duration of 5 minutes to 100 hours, preferably 1 to 10 hours and more preferably 2 to 8 hours.

According to a particularly preferred embodiment of process 1 according to the present invention it is also advantageous that the oxygen content of the solution or dispersion provided in process step α) is less than 1,000 ppb, preferably less than 500 ppb, more preferably less than 100 ppb, more preferably less than 10 ppb, more preferably less than 1 ppb, more preferably less than 0.5 ppb and most preferably less than 0.25 ppb, in each case based on the total weight of the solution or dispersion. According to a particularly preferred embodiment of process 1 according to the present invention the solution or dispersion provided in process step α) is completely free of any oxygen (i. e. the oxygen content is 0 ppb).

There are different approaches of adjusting the oxygen content in the solution or dispersion that is provided in process step α) and also to maintain this low oxygen content during the polymerization reaction in process step β).

According to one approach the solution or dispersion provided in process step α) (or the liquid components that are used to prepare the solution or dispersion) can be degassed, for example by introducing an inert gas such as N₂, Argon, CO₂ or a mixture thereof into the solution or dispersion provided in process step α) to reduce the initial oxygen content in the solution or dispersion. Alternatively, the solution or dispersion provided in process step α) (or the liquid components that are used to prepare the solution or dispersion) can be subjected to a treatment with a reduced pressure in order to reduce the initial oxygen content, for example by stirring the solution or dispersion while applying a vacuum, or can be subjected to a treatment with ultra sound or can be subjected to a combination of a treatment with a reduced pressure and a treatment with ultra sound.

In order to ensure that the low oxygen content is maintained during the polymerization reaction in process step β) it may be advantageous to perform the polymerization reaction under an inert gas atmosphere, preferably under a N₂-atmosphere, under a COz-atmosphere, under an argon atmosphere or under an atmosphere of a mixture of at least two of these inert gases, wherein it may also be advantageous that the oxidative polymerization in process step β) is performed under a pressure that is equal to or above the vapor pressure of the solution or dispersion during the polymerization reaction in process step β). Preferably, the oxidative polymerization in process step β) is performed under a pressure that is at least 0.1 mbar, more preferably at least 0.5 mbar and most preferably at least 1 mbar above the vapor pressure of the solution or dispersion during the polymerization reaction in process step β). To ensure that the low oxygen content is maintained during the polymerization reaction in process step β) it is also possible to perform the oxidative polymerization in process step β) under a reduced pressure, preferably under a pressure of not more than 0.8 bar and most preferably under a pressure of not more than 0.5 bar.

### Process for producing a layered body, preferably a capacitor

The layer bodies according to the present invention that comprise a conductive layer based on a polymer having the general formula (III) can be prepared by means of process 2 according to the invention, this process comprising the process steps:
**A)** providing a substrate;
**B)** applying the liquid composition according to the invention, preferably a liquid composition according to any of its 1^{st} to the 6^{th} embodiment, onto a surface of the substrate;
**C)** at least partially removing the dispersant **i)** for the formation of a conductive layer that at least partially covers a surface of the substrate.

According to a preferred embodiment of that process, the substrate is a porous electrode body made of an electrode material, wherein a dielectric layer at least partially covers a surface of this electrode material and wherein the electrically conductive layer is a solid electrolyte layer or part of a solid electrolyte layer that at least partially covers a surface of the dielectric layer. In case of such a substrate, process 2 according to the invention preferably comprises the steps of:
**A)** provision of a porous electrode body made of an electrode material, wherein a dielectric layer at least partially covers a surface of this electrode material;
**B)** introduction of the liquid composition according to the invention, preferably a liquid composition according to any of its 1^{st} to the 6^{th} embodiment, into at least a part of the porous electrode body provided in process step **A)** so as to apply the dispersion onto at least a part of the surface of the porous electrode body, preferably onto at least a part of the surface of the dielectric layer;
**C)** at least partial removal of the dispersant **i)** for the formation of a solid electrolyte that at least partially covers a surface of the dielectric layer;
**D)** optionally application of a dispersion containing a conductive polymer, preferably a dispersion comprising a polythiophene and a dispersant, more preferably a dispersion comprising a dispersant and particles of a complex of a polythiophene and a polymeric anion, even more preferably a PEDOT/PSS-dispersion, onto at least a part of the surface of the solid electrolyte layer and at least partial removal of the dispersant for the formation of a polymeric outer layer that at least partially covers a surface of the solid electrolyte layer;
**E)** optionally filling at least a part of the pores of the porous electrode body obtained in process step **C)** or in process step **D)** (*i. e.*, the porous electrode body in which the surface of the dielectric layer is at least partially covered with a solid electrolyte layer) with an impregnation solution comprising at least one impregnation solvent.

### Process step A):

In process step **A)** a porous electrode body made of an electrode material is provided, wherein a dielectric layer at least partially covers a surface of this electrode material.

In principle, the porous electrode body can be manufactured in that a valve-metal powder with a high surface area is pressed and sintered to form a porous electrode body. In this context, an electrical contact wire preferably made from a valve metal, such as tantalum, is conventionally pressed into the porous electrode body. The porous electrode body is then coated with a dielectric, that is, an oxide layer, for example, by electrochemical oxidation. As an alternative, metal films can be etched and coated with a dielectric by electrochemical oxidation in order to obtain an anode film with a porous region. In the case of a wound capacitor, an anode film with a porous region, which forms the electrode body, and a cathode film are separated by separators and wound up.

Within the scope of the invention, metals of which the oxide coatings do not allow the flow of current uniformly in both directions are understood as valve metals. With a voltage applied at the anode, the oxide layers of the valve metals block the flow of current, while in the case of a voltage applied to the cathode, considerable currents occur, which can destroy the oxide layer. The valve metals include Be, Mg, Al, Ge, Si, Sn, Sb, Bi, Ti, Zr, Hf, V, Nb, Ta and Wand an alloy or compound of at least one of these metals with other elements. The most well-known representatives of the valve metals are Al, Ta and Nb. Combinations of electrical properties comparable with a valve metal are those with metallic conductivity, which can be oxidised and of which the oxide layers provide the properties described above. For example, NbO shows metallic conductivity, but is not generally regarded as a valve metal. However, layers of oxidised NbO show the typical properties of valve-metal oxide layers, so that NbO or an alloy or compound of NbO with other elements are typical examples of such compounds with electrical properties comparable with a valve metal. Electrode materials made of tantalum, aluminium and such electrode materials which are based on niobium or niobium oxide are preferred. Aluminium and tantalum are particularly preferred as an electrode material.

For the manufacture of the porous electrode body often with a porous region, the valve metals can be sintered, for example, in powdered form to provide a generally porous electrode body, or alternatively, a porous structure is imprinted onto a metallic body. The latter can be implemented, for example, by etching a film.

In the following, for the sake of simplicity, bodies with a porous region are also referred to as porous. For example, electrode bodies with a porous region are also referred to as porous electrode bodies. On the one hand, the porous bodies can be penetrated by a plurality of channels and therefore be sponge-like. This is often the case if tantalum is used in the construction of the capacitor. On the other hand, pores can be present only at the surface, and the region disposed below the surface pores can be formed in a solid manner. This is often observed, if aluminium is used in the capacitor construction.

The porous electrode bodies manufactured in this manner are then oxidised, for example in an appropriate electrolyte, for example, phosphoric acid or an aqueous solution of ammonium adipate, by applying a voltage, in order to form the dielectric. The magnitude of this forming voltage is dependent upon the oxide-layer thickness to be achieved or respectively the subsequent operating voltage of the capacitor. Preferred forming voltages lie in a range of from 1 to 1000 V, particularly preferably in a range of from 10 to 200 V, more particularly preferably in a range of from 15-100 V, more preferably in a range of from 20-50 V.

The porous electrode bodies used preferably have a porosity of 10 to 90 %, preferably of 30 to 80 %, particularly preferably of 50 to 80 %, and an average pore diameter of from 10 to 10000 nm, preferably from 50 to 5000 nm, particularly preferably from 100 to 3000 nm.

According to a special embodiment of the process according to the invention, the electrolytic capacitor to be manufactured is an aluminium wound capacitor. In this case, in process step **A),** a porous aluminium film as electrode material is formed anodically, whereby an aluminium-oxide coating is formed as the dielectric. The aluminium film (anode film) thus obtained is then provided with a contact wire and wound up with a further porous aluminium film (cathode film) also provided with a contact wire, whereby these two films are separated from one another by one or more separator papers, which are based, for example, on cellulose or preferably on synthetic papers. After the winding, the anode bodies obtained in this manner are fixed, for example, by means of an adhesive tape. The separator paper or papers can be carbonised by heating in an oven. This manner of manufacturing anode bodies for aluminium wound capacitors is sufficiently known from the prior art and is described, for example in US 7,497,879 B2.

### Process steps B) and C):

In process step **B)** of process 2 according to the invention, the liquid composition according to the present invention, preferably the liquid composition according to any of its 1^{st} to the 6^{th} embodiment, is introduced into at least a part of the porous electrode body provided in process step **A).** In process step **C)** the dispersant **i)** is then at least partially removed for the formation of a solid electrolyte layer that at least partially covers a surface of the dielectric layer.

The liquid composition can, besides the polymer **ii)** of the general formula (**III**) and the dispersant i), comprise further additives, such as surface-active substances, e.g. anionic surfactants, such as e.g. alkylbenzenesulphonic acids and salts, paraffin sulphonates, alcohol sulphonates, ether sulphonates, sulphosuccinates, phosphate esters, alkyl ether carboxylic acids or carboxylates, cationic surfactants, such as e.g. quaternary alkylammonium salts, nonionic surfactants, such as e.g. linear alcohol ethoxylates, oxo alcohol ethoxylates, alkylphenol ethoxylates or alkyl polyglucosides, in particular surfactants that are commercially available under the trademarks Dynol^{™} and Zonyl^{™} or adhesion promoters, such as e.g. organofunctional silanes or hydrolysates thereof, e.g. 3-glycidoxypropyltrialkoxysilane, 3-amino-propyl-triethoxysilane, 3-mercaptopropyltrimethoxysilane, 3-methacryloxypropyltrimethoxy-silane, vinyltrimethoxysilane or octyltriethoxysilane, crosslinking agents, such as melamine compounds, masked isocyanates, functional silanes - e.g. tetraethoxysilane, alkoxysilane hydrolysates, e.g. based on tetraethoxysilane, epoxysilanes, such as 3-glycidoxypropyltrialkoxysilane - polyurethanes, polyacrylates or polyolefin dispersions. The above-mentioned adhesion promoters can also be applied onto the porous electrode body, preferably onto the dielectric layer, before applying the conductive polymer according to the invention.

The liquid composition can comprise further additives which optionally increase the conductivity, such as e.g. compounds containing ether groups, such as e.g. tetrahydrofuran, compounds containing lactone groups, such as γ-butyrolactone, γ-vaterotactone, compounds containing amide or lactam groups, such as caprolactam, N-methylcaprolactam, N,N-dimethylacetamide, N-methylacetamide, N,N-dimethylformamide (DMF), N-methylformamide, N-methylformanilide, N-methylpyrrolidone (NMP), N-octylpyrrolidone, pyrrolidone, sulphones and sulphoxides, such as e.g. sulpholane (tetramethylene sulphone), dimethylsulphoxide (DMSO), sugars or sugar derivatives, such as e.g. sucrose, glucose, fructose, lactose, sugar alcohols, such as e.g. sorbitol, mannitol, furan derivatives, such as e.g. 2-furancarboxylic acid, 3-furancarboxylic acid, glycerol, diglycerol, triglycerol or tetraglycerol, glycols such as ethylene glycol, diethylene glycol, polyethylene glycol, propylene glycol, dipropylene glycol or polypropylene glycol.

The liquid composition can moreover comprise as a further additive one or more organic binders which are soluble in water or water-miscible solvents. Moreover, polymer composition 2 may also comprise as a further additive one or more compounds which increase the heat stability of the electrically conductive layers, such as e.g. aromatic compounds with at least two OH groups, such as e.g. gallic acid (3,4,5-trihydroxybenzoic acid), tannin or flavonoids.

The liquid composition is introduced into the porous region using known processes, for example, immersion, dipping, pouring, dripping, injection, spraying, spreading, painting or printing, for example, ink-jet printing, screen printing or tampon printing. The introduction is preferably carried out in that the porous electrode body provided in process step **A)** is immersed in the liquid composition and is accordingly impregnated with this liquid composition. The immersion into or the impregnation with the liquid composition is preferably implemented for a duration in a range of from 1 second to 120 min, particularly preferably in a range of from 10 seconds to 60 min and most preferably in a range of from 30 seconds to 15 min. The introduction of the liquid composition into the anode body can be facilitated, for example, by increased or reduced pressure, vibration, ultrasound or heat.

After the porous electrode bodies have been impregnated with the liquid composition as described above, the dispersant i) is at least partially removed in process step **C),** so that a solid electrolyte layer is formed, which covers the dielectric partially or completely. In this context, it is preferable that the covering of the dielectric by the solid electrolyte layer preferably amounts to at least 50 %, particularly preferably at least 70 % and most preferably at least 80 %, wherein the measurement of the capacitance of the capacitor in a dry and wet condition at 120°C allows a determination as described in DE-A-10 2005 043 828.

The removal of the dispersant **i)** is preferably carried out in that the porous electrode body is removed from the liquid composition used in process step **B)** and is subsequently dried, wherein the drying preferably takes place at a temperature in a range from 20°C to 200°C, particularly preferably in a range from 50°C to 180°C and more preferably in a range from 80°C to 150°C. The drying conditions (*i. e*. drying time, drying pressure and drying temperature) are preferably adjusted to be within a range that ensures that at least 50 wt.-%, more preferably at least 75 wt.-%, even more preferably at least 90 wt.-%, even more preferably at least 95 wt.-% and most preferably at least 99 wt.-% of the total amount of the dispersant **i)** is removed when forming the solid electrolyte layer. In a particularly preferred embodiment of the process according to the present invention the drying conditions are adjusted to be within a range that ensures that the dispersant **i)** is completely removed when forming the solid electrolyte layer.

Process steps **B)** and **C)** can be repeated several times depending on the desired thickness of the conductive layer that is based on the polymers of the general formula (**III**).

After the conductive layer based on the polymers according to the present invention carrying sulfate groups has been applied to the dielectric layer (or onto intermediate layers such as adhesive layers that may have been applied previously) for the formation of a solid electrolyte layer, additional conductive layers can be applied, for example based on foreign-doped conductive polymers such as PEDOT/PSS, to from additional part-layers of the solid electrolyte layer. The disclosure of WO 2014/048562 regarding the combined use of self-doped polymers, like PEDOT-S, and foreign-doped polymers, like PEDOT/PSS, for the formation of a solid electrolyte is incorporated herein by reference and forms a part of the disclosure of the present application.

### Process step D):

In process step **D)** a dispersion containing a conductive polymer, preferably a dispersion comprising a polythiophene and a dispersant, more preferably a dispersion comprising a dispersant and particles of a complex of a polythiophene and a polymeric anion, even more preferably a PEDOT/PSS-dispersion, may be applied onto at least a part of the surface of the solid electrolyte layer, followed by an at least partial removal of the dispersant for the formation of a polymeric outer layer that at least partially covers the surface of the solid electrolyte layer (as disclosed, for example, in DE 10 2004 022 674 A1). The term *"polymeric outer"* layer as used herein preferably refers to an outer layer that, although it may comprise the very same conductive polymer as the solid electrolyte layer, nevertheless differs from the solid electrolyte layer with respect to, for example, the chemical composition, the conductivity and/or a property such as the hardness, the surface roughness, the adhesion property *etc..* The polymeric outer layer, usually having a thickness in the range from 5 to 50 µm, serves as a mechanical buffer between the capacitor anode and the cathode-side contact, preventing the cathode-side-contact to come into contact with the dielectric under mechanical stresses that may occur during the manufacture of the capacitor.

Before applying a dispersion containing a conductive polymer onto at least a part of the surface of the solid electrolyte layer in process step **D),** it may also be advantageous to apply a crosslinker onto at least a part of the surface of the solid electrolyte layer in order to improve the coverage of the capacitor anode by the polymeric outer layer. Suitable crosslinkers and processes for their application are, for example, disclosed in DE 10 2009 007 594 A1.

### Process step E):

In process step **E)** of the process according to the invention at least a part of the pores of the porous electrode body obtained in process step **C)** (*i. e*., the porous electrode body in which the dielectric layer is at least partially covered with a solid electrolyte layer) can be filled with an impregnation solution comprising at least one impregnation solvent.

The immersion in or respectively the impregnation with the impregnation solution is preferably implemented for a duration in a range of from 1 second to 120 min, particularly preferably in a range of from 10 seconds to 60 min and most preferably in a range of from 30 seconds to 15 min. The immersion is preferably carried out in that the porous electrode body obtained in process step **C)** is immersed at least partially in the impregnation solution or the impregnation solution is injected into the porous electrode body and is accordingly impregnated with this impregnation solution. The introduction of the impregnation solution into the porous electrode body can be facilitated, for example, by increased or reduced pressure, vibration, ultrasound or heat.

Impregnation solutions may include one or more compounds which increase the heat stability of the electrically conductive layers, such as e.g. aromatic compounds with at least two OH groups, such as e.g. gallic acid (3,4,5-trihydroxybenzoic acid), tannin or flavonoids. When introducing an impregnation solution comprising such stabilizers into the pores of the electrode body, it is also advantageous to perform a drying step afterwards.Examples of the impregnation solvent include water, a sulfone compound, a lactone compound, a carbonate compound, and a polyhydric alcohol. The impregnation solvent may be used singly or in combination of two or more kinds thereof.

Examples of the sulfone compound include sulfolane, dimethyl sulfoxide, and diethyl sulfoxide. Examples of the lactone compound include γ-butyrolactone and γ-valerolactone. Examples of the carbonate compound include dimethyl carbonate, diethyl carbonate, ethyl methyl carbonate, ethylene carbonate, propylene carbonate, and fluoroethylene carbonate. Examples of the polyhydric alcohol include a glycerin compound, a sugar alcohol compound, and a glycol compound. Examples of the glycerin compound include glycerin, polyglycerin (diglycerin, triglycerin, etc.), and derivatives thereof. Examples of the glycol compound include an alkylene glycol (C2-4 alkylene glycol (ethylene glycol, propylene glycol, etc.), and the like), a polyalkylene glycol (poly C2-4 alkylene glycol (diethylene glycol, dipropylene glycol, triethylene glycol, polyethylene glycol, etc.), and the like).

The impregnation solution may include a solute. Examples of the solute include an acid component, such as carboxylic acids, sulfur-containing acids, boron-containing acids and phosphorus-containing acids, and a base component, such as ammonia, amine, a quaternary ammonium compound and an amidinium compound.

The liquid component may include the acid component and the base component in a free state or in a salt form, respectively. The liquid component may include an organic salt. Examples of the organic salt include those in which at least one of the acid component and the base component is organic.

The concentration of the solute in the liquid component is, for example, in a range from 0.1 to 25 wt.-%, inclusive, and may be in a range from 0.5 to 15 wt.-%, inclusive.

### Encapsulation in process step F):

After in process step **C)** at least a part of the dispersant **i)** has been removed or - if process step **D)** and/or process step **E)** are performed - after in process step **D)** a polymeric outer layer has been applied or after in process step **E)** at least a part of the pores of the porous electrode body obtained in process step **C)** has been filled with an impregnation solution, the electrolytic capacitors can be finished, particularly encapsulated, in a manner known to the person skilled in the art. In the case of a tantalum electrolytic capacitor, the capacitor body can be coated, for example, with a graphite layer and a silver layer, as known from DE-A-10 2005 043 828, while in the case of an aluminium wound capacitor corresponding to the teaching of US 7,497,879 B2, the capacitor body is built into an aluminium cup, provided with a sealing rubber and firmly closed in a mechanical manner by flanging.

Encapsulation is preferably accomplished by sealing the capacitor body with resins, such as epoxy resins or thermoplastic resins like those disclosed in EP 0 447 165 A2. In case of an aluminium electrolytic capacitor encapsulation is preferably accomplished by providing the porous electrode body obtained in process step e) with the aluminium cup and closing it with the sealing rubber.

The features disclosed in the claims, the specification, and the drawings maybe essential for different embodiments of the claimed invention, both separately and in any combination with each other.

### DESCRIPTION OF THE DRAWINGS

The following schematic drawings show aspects of the invention for improving the understanding of the invention in connection with some exemplary illustrations.

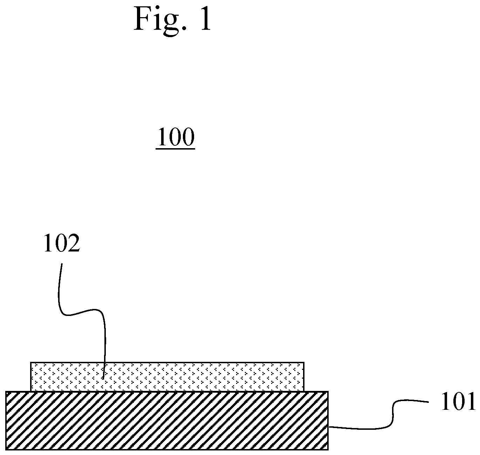

Figure 1 shows the structure of a layer body 100 prepared by the process for the preparation of a layered structure according to the invention, for example an antistatic film, in general form. On the substrate surface of a substrate 101, in the case of an antistatic film often a PE, PP or PET layer, is an electrically conductive layer 102 that has been prepared with the composition according to the invention.

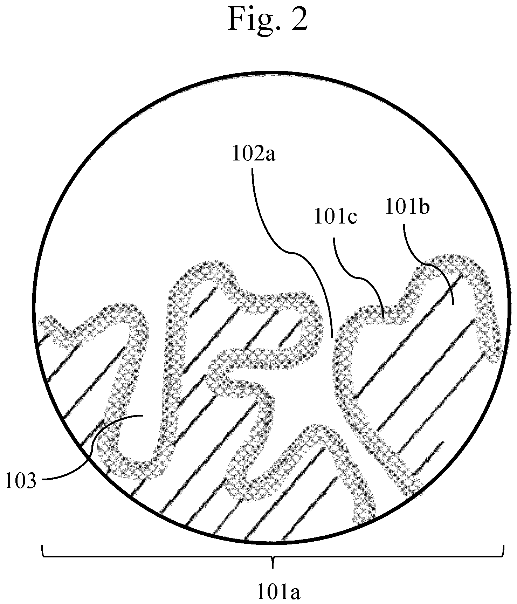

Fig. 2 shows a schematic sectional view through a part of a capacitor obtained by a particular embodiment of the process for the preparation of a layered structure according to the invention. The capacitor comprises a porous electrode body 101a comprising pores 103, made mostly from a porous electrode material 101b such as aluminium or tantalum. On the surface of the electrode material 101b, a dielectric layer 101c is formed as a thin layer, so that an anode body is formed which is still porous, comprising the electrode body 101a made of the electrode material 101b and the dielectric layer 101c. On the dielectric layer 101c, there follows, optionally after further layers, a layer of a solid electrolyte layer 102a (that comprises the electrically conductive polymer of the general formula (**III**) according to the present invention), so that a capacitor body is formed, comprising the electrode body 101a made of the electrode material 101b, the dielectric layer 101c and the solid electrolyte layer 102a.

### MEASUREMENT METHODS:

### Determination of the content of unsaturated organic sulfonic acids by means of ¹H NMR

The content of unsaturated organic sulfonic acids, particularly the content of sodium monoethenyl ester sulfate (Ethenyl-OSNa) in compositions of thiophene monomers was determined by means of ¹H NMR via the presence of characteristic peaks. Table 1 shows these characteristic peaks for Ethenyl-OSNa.

**Table 1: ¹H NMR signals (D₂O) of Ethenyl-OSNa:**

| **proton identity** | **¹H NMR shift** / **ppm** | **proton count** |
|---|---|---|
| **1** | 4.55 | 2H |

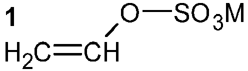

The integration of EDOT-OSNa and ProDOT-OSNa aromatic thiophene protons (6.53 and 6.75 ppm, respectively) and those of terminal alkene protons (**1**) allowed determination of the respective three component molar ratio (EDOT-OSNa, ProDOT-OSNa and Ethenyl-OSNa).

### Conductivity

A cleaned glass substrate of size 50 mm × 50 mm was laid on a spin coater and 10 ml of the liquid composition according to the invention was distributed over the substrate. The remaining solution was then spun off by rotation of the plate. Thereafter, the substrate thus coated was dried for 15 minutes at 130°C on a hot plate. The layer thickness was then determined by means of a layer thickness measuring device. (Tencor, Alphastep 500). The conductivity was determined in that Ag electrodes of 25 mm length were vapor deposited at a distance of 10 mm via a shadow mask. The surface resistance determined with an electrometer (Keithly 614) was multiplied by the layer thickness in order to obtain the specific electrical resistivity. The conductivity is the inverse of the specific electrical resistivity.

### Equivalent series resistance (ESR)

The equivalent series resistance (in mΩ) of a capacitor was determined at 20°C at 100 kHz by means of an LCR meter (Agilent 4284A). In each capacitor experiment 3 capacitors have been prepared and the average ESR-value was determined.

### Average

If not otherwise mentioned, the average corresponds to the arithmetical average value.

### Solid content

The solid content was determined by gravimetry using a precision scale (Mettler AE 240). First the empty weighing bottle including lid is weighed (Weight A). Then 3 g of dispersion to be analysed is filled into the bottle, closed by the lid and weighed again to determine the exact total weight (weight B). The bottle is placed in a drying oven with ventilation (Memmert UNB200) at 100 °C for 16 hours. When the sample bottle is removed from the oven, immediate coverage by the glass lid is important due to the hygroscopic nature of the dry dispersion material. After 10 - 15 min of cooling down period the bottle is weighed again including lid to determine weight C. There are always at least 2 further repeats conducted to allow determine of an average solid content.

### Thermal Stability investigations

A cleaned glass substrate of size 50 mm × 50 mm was laid on a spin coater and 10 ml of the liquid composition according to the invention was distributed over the substrate. The remaining solution was then spun off by rotation of the plate. Thereafter, the substrate thus coated was dried for 15 minutes at 130°C on a hot plate. The conductivity was determined in that Ag electrodes of 25 mm length were vapor deposited at a distance of 10 mm via a shadow mask. The initial surface resistance (Ω) was then determined with a multimeter (METRAHIT 12S). The coated glass substrate was incubated in an oven at 150°C for 6 days. Every 24 h, excluding weekends, surface resistance measurements were conducted. Glass substrates were removed from the oven for 30 mins prior to measurement to allow cooling to room temperature. The layer thickness was then determined by means of a layer thickness measuring device (Tencor, Alphastep 500).

### EXAMPLES

EDOT-MeOH (CAS#: 146796-02-3), the precursor compound for the preparation of EDOT-OSNa, was purchased from Synmax Biochemical Co., Ltd. (Taiwan) with a purity of > 99% and was directly applied to the following syntheses:

### Example 1: synthesis of EDOT-OSNa (in toluene with acetone precipitation)

Sodium hydride (7.11 g, 178 mmol, 60% oil dispersion) was added to dry toluene (45 mL) and placed under a nitrogen atmosphere and with continuous stirring. EDOT-MeOH (15.00 g, 87 mmol) dissolved in toluene (75 mL) was slowly added with vigorous stirring. The reaction mixture was heated to reflux for 1 h. Then ethylene sulfate (11.9 g, 96 mmol) dissolved in tetrahydrofuran (30 mL) and then toluene (150 mL) was slowly added dropwise to the stirring solution. The reaction was refluxed for a further 2 h and cooled to room temperature. Acetone (200 mL) was added under vigorous stirring. The resulting suspension was filtered through a fritted filter and washed with additional acetone under a nitrogen atmosphere. The product was dried at 40°C under vacuum to obtain *"Sample #1"* as an orange/yellow powder (14.7 g, 46 mmol, 53%).

¹H NMR: (600 MHz, D2O) δ: 6.54 (2H, m), 4.46 (1H, m), 4.32 (1H, m), 4.22 (2H ,t), 4.13 (1H, m), 3.89-3.81 (4H, m) ppm. ¹³C NMR: (150 MHz, D2O) δ: 140.6, 140.4, 100.5, 100.3, 72.8, 69.5, 69.0, 67.7, 65.5 ppm.MS (negative mode): calculated for C₉H₁₂O₇S₂ m/z: 296.00; detected [M-H]⁻: 294.99.

For Example 1, the resulting product mixture ratio of EDOT-OSNa : Ethenyl-OSNa was about 90 : 10 (as determined by ¹H NMR).

### Example 2: synthesis of EDOT-OSNa (in tetrahydrofuran with acetone precipitation)

Sodium hydride (4.74 g, 118 mmol, 60% oil dispersion) was added to tetrahydrofuran (25 mL) and placed under a nitrogen atmosphere and with continuous stirring. EDOT-MeOH (10.00 g, 58 mmol) dissolved in tetrahydrofuran (40 mL) was slowly added with vigorous stirring. The reaction mixture was heated to reflux for 1 h. Then ethylene sulfate (7.93 g, 64 mmol) dissolved in tetrahydrofuran (30 mL) was slowly added dropwise to the stirring solution. The reaction was refluxed for a further 3 h and cooled to room temperature. Ethanol (15 mL) was added under vigorous stirring and the resulting solution was concentrated under vacuum to remove THF. Acetone (50 mL) was added and stirring vigorously to generate a suspension. The resulting suspension was filtered through a fritted filter and washed with additional acetone under a nitrogen atmosphere. The product was dried at 40°C under vacuum to obtain *"Sample #2"* as a beige powder (13.6 g, 43 mmol, 73%).

For Example 2, the resulting product mixture ratio of EDOT-OSNa : Ethenyl-OSNa was about 93 : 7 (as determined by ¹H NMR). The analytical data conformed to that reported for Example 1.

### Example 3: synthesis of EDOT-OSNa (in tetrahydrofuran with additional iso-propanol precipitation)

Sodium hydride (9.48 g, 237 mmol, 60% oil dispersion) was added to tetrahydrofuran (50mL) and placed under a nitrogen atmosphere and with continuous stirring. EDOT-MeOH (20.00 g, 116 mmol) dissolved in tetrahydrofuran (80 mL) was slowly added with vigorous stirring. The reaction mixture was heated to reflux for 1 h. Then ethylene sulfate (15.9 g, 128 mmol) dissolved in tetrahydrofuran (70 mL) was slowly added dropwise to the stirring solution. The reaction was refluxed for a further 3 h and cooled to room temperature. Ethanol (40 mL) and acetone (100 mL) was added under vigorous stirring and a fine suspension was observed, and filtered over a fritted filter. The filtrate was taken and dropwise added to propanol (1 L) at room temperature. The resulting suspension was filtered through a fritted filter and washed with additional propanol. The product was dried at 40°C under vacuum to obtain *"Sample #3"* as a beige powder (15.7 g, 49 mmol, 42%).

For Example 3, the resulting product mixture ratio of EDOT-OSNa : Ethenyl-OSNa was about 96 : 4 (as determined by ¹H NMR). The analytical data conformed to that reported for Example 1.

### Further Derivatisations

In the following examples, EDOT-MeOH was reacted with isomers of propylene sulfate, instead of ethylene sulfate.

### Example 4: synthesis of EDOT-OS derivative from 1,2-propylene sulfate

Sodium hydride (6.87 g, 172 mmol, 60% oil dispersion) was added to tetrahydrofuran (100 mL) and placed under a nitrogen atmosphere and with continuous stirring. EDOT-MeOH (28.20 g, 163 mmol) dissolved in tetrahydrofuran (100 mL) was slowly added with vigorous stirring. The reaction mixture was heated to reflux for 1 h. Then 1,2-propylene sulfate (25.0 g, 172 mmol) dissolved in tetrahydrofuran (100 mL) was slowly added dropwise to the stirring solution. The reaction was refluxed for a further 1 h and cooled to room temperature, and then cooled with an ice-water bath to ca. 0 °C. Iso-propanol (300 mL) was added slowly under vigorous stirring. The resulting suspension was filtered through a fritted filter. The product was dried at 40°C under vacuum to obtain *"Sample #4"* as a white-grey powder (40.9 g, 123 mmol, 75%).

For Example 4, the resulting product mixture ratio of EDOT-OSNa derivative : 1-propen-2-ol, 2-(sodium sulfate) was about 75 : 25 (as determined by ¹H NMR).

### Example 5: synthesis of EDOT-OS derivative from 1,3-propylene sulfate

Sodium hydride (11.8 g, 296 mmol, 60% oil dispersion) was added to tetrahydrofuran (100 mL) and placed under a nitrogen atmosphere and with continuous stirring. EDOT-MeOH (25.0 g, 145 mmol) dissolved in tetrahydrofuran (100 mL) was slowly added with vigorous stirring. The reaction mixture was heated to reflux for 1 h. Then 1,3-propylene sulfate (24.6 g, 160 mmol) dissolved in tetrahydrofuran (100 mL) was slowly added dropwise to the stirring solution. The reaction was refluxed for a further 3 h and cooled to room temperature, and then cooled with an ice-water bath to ca. 0 °C. Iso-propanol (300 mL) was added slowly under vigorous stirring. The resulting suspension was filtered through a fritted filter. The product was dried at 40°C under vacuum to obtain *"Sample #5"* as a white-orange powder (35.7 g, 108 mmol, 74%).

For Example 5, the resulting product mixture ratio of EDOT-OSNa derivative : 2-propen-1-yl sodium sulfate was about 50 : 50 (as determined by ¹H NMR).

### Example 6: polymerization of monomer produced in Examples 1-5

The product mixture as obtained in Examples 1-5 (4.82 g, 15 mmol) was dissolved in dist. water (25 mL) and was degassed using nitrogen for 60 min, whilst cooling to 10°C. Iron(II)sulfate (1.11 g, 4 mmol) was dissolved in dist. water (5 mL) and degassed using nitrogen for 30 min. The iron(ll)sulfate solution was then added in one portion to the EDOT-OS Na solution (or the solution of the EDOT-OS derivative). Then DMSO (30 mg, 0.38 mmol) was added to the solution too. Sodium persulfate (4.92 g, 20.6 mmol) was added to dist. water (10 mL) and degassed with nitrogen for 60 min. The reaction solution was stirred throughout, and the temperature was maintained at 10°C for the entirety of the slow addition of the sodium persulfate solution. The reaction ran for 17 h with warming to room temperature, whereafter the solution was added to ion exchangers (Lewatit^{®} S108H and Lewatit^{®} MP 62) and the mixture was stirred at room temperature for 30 min. The process was repeated 3 times with a filtration after every round. After the ion exchangers had been filtered off, the solution was diluted to 1.0-3.0% solids content and subsequently ultrasonic treated to yield a dark blue polymer solution.

**Table 2: thin film conductivities and solid contents of dispersions produced in Example 6**

| sample # used to prepare PEDOT-OS (or polymer of derivative of EDOT-OS) | EDOT-OS (or EDOT-OS derivative) : alkenyl-OS molar ratio | solids content [wt.-%] | conductivity [S/cm] |
|---|---|---|---|
| 1 | 90 : 10 | 1.00 | 2 |
| 2 | 93 : 7 | 1.20 | 135 |
| 3 | 96 : 4 | 1.00 | 314 |
| 4 | 75 : 25 | 2.50 | 21 |
| 5 | 50 : 50 | 2.80 | 5 |

Table 2 shows the effect of % molar alkene side-product content (Ethenyl-OSNa) on the conductivity. As can be seen, reducing the content of that by-product in the monomer leads to higher conductivities in the conductive layer. Similarly different side-chain structures generate alkenyl side-products which also result in low conductivities after polymerization, i.e. those derived from Samples #4-5

### Example 7: thermal stability investigations

The thermal stability of different self-doped thiophene-based conductive polymers were then tested at 150 °C for 6 days having been deposited as thin films on glass substrates. Two types of self-doped materials were tested: firstly, PEDOT-SNa (sulfonic acid functionalized; prior art and not according to the invention), and PEDOT-OSNa (O-sulfate functionalized polymer; according to the invention as prepared in Example 4, based upon the monomer as obtained in Example 3). The chemical structures of the thiophene-based intrinsically conducting polymers used in this example are shown below. All the dispersions tested contained 1% solids content.

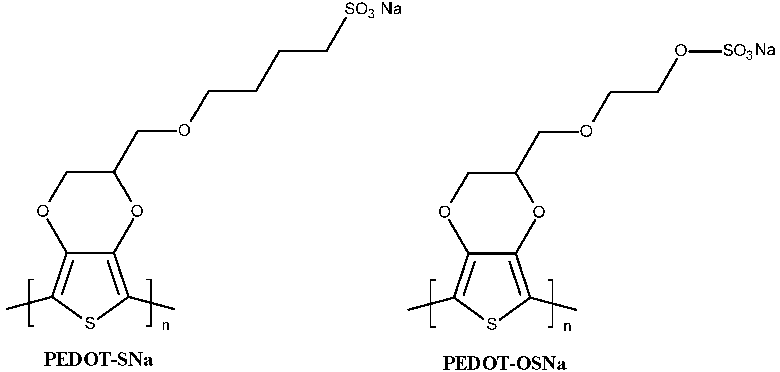

The formation of thin films using 1% solids content dispersions generated thicknesses between 110-130 nm (Table 3). Exposure of the coated substrates in a heating oven at 150 °C over 6 days lead in all cases to an increase in surface resistance. The results of the time-dependent measurement of the surface resistance is shown in Table 4.

In the case of sulfonic acid-functionalized PEDOT-SNa, after 24 h heating a near 20-fold increase in the surface resistance from 580 to 11,140 Ω was observed. A further 48 h of heat exposure an additional near 30-fold increase of the relative surface resistance occurred. After 6 days surface resistance was measured at 6,000,000 Ω, which is an over 10,000-fold increase relative to the initial surface resistance. These results indicate a clear degradation of the thin film's surface resistance.

For PEDOT-OSNa, a similar starting surface resistance of 536 Ω was measured, which after exposure to 150 °C for 24 h lead to a 3-fold increase in surface resistance. Over 6 days, at every time-point, increases in surface resistance were measured until the last measured surface resistance of 100,667 Ω, which is a 188-fold increase relative to the initial surface resistance.

When comparing the PEDOT-SNa- and PEDOT-OSNa-compositions, it is evident that both dispersions are thermally unstable from a similar starting point. However, PEDOT-SNa shows across all measured time-points an increased instability relative to PEDOT-OSNa.

**Table 3: self-doped conductive polymer dispersions and initial thin film properties**

| material | solids content [wt.-%] | surface resistance (t = 0 h) / Ω | sheet thickness / nm |
|---|---|---|---|
| PEDOT-SNa | 1.0 | 580 | 126 |
| PEDOT-OSNa | 1.0 | 536 | 116 |

**Table 4: Thermal stability results of conductive polymer thin films tabulating their surface resistance (Ω) after exposure at 150 °C in air over 6 days.**

| material | PEDOT-SNa | | PEDOT-OSNa | |
|---|---|---|---|---|
| SR / Ω¹ | Absolute | Relative | Absolute | Relative |
| t = 0 h | 580 | 1 | 536 | 1 |
| 24 h | 11,140 | 19 | 1,841 | 3 |
| 48 h | 60,133 | 104 | 3,840 | 7 |
| 72 h | 330,333 | 570 | 7,930 | 15 |
| 144 h | 6,000,000 | 10345 | 100,667 | 188 |

| | | | | |
|---|---|---|---|---|
| ¹⁾: Absolute surface resistance was measured in Ohm (Ω) and relative values are given relative to t = 0 h | | | | |

### Example 8

### Process step A):

Tantalum powder having a specific capacitance of 30,000 CV/g was pressed to pellets with inclusion of a tantalum wire and sintered to form a porous electrode body having dimensions of 1.4 mm × 2.9 mm × 4.0 mm. The porous electrode bodies were anodized in a phosphoric acid electrolyte at 60 V to form a dielectric, to obtain capacitor bodies.

### Process steps B) and C):

A PEDOT-SNa solution was prepared by stirring into 10 g of the PEDOT-SNa solution used in "Example 7", 0.01 g of 3-glycidoxypropyltrimethoxysilane and 0.01 g of surfactant Dynol 604.

A PEDOT-OSNa solution was prepared by stirring into 10 g of the PEDOT-OSNa used in "Example 7" 0.01 g of 3-glycidoxypropyltrimethoxysilane and 0.01 g of surfactant Dynol 604.

One set of capacitors obtained in process A) were dipped into the thus prepared PEDOT-SNa solution. Thereafter, drying was carried out at 120 °C for 10 min. This process of dipping and drying was repeated for additional 5 times.

Another set of capacitors obtained in process A) were dipped into the thus prepared PEDOT-OSNa solution. Thereafter, drying was carried out at 120 °C for 10 min. This process of dipping and drying was repeated for additional 5 times.

### Process steps D):

Subsequently, both sets of capacitor bodies of process C) of were dipped into a PEDOT/PSS dispersion (Clevios K Nano LV, Heraeus Epurio). Thereafter, drying was carried out at 120 °C for 10 min. The dipping and drying were carried out three further times.

Subsequently, each set of capacitor bodies were impregnated in a crosslinker agent solution containing diaminodecane toluene sulfonate salt dissolved in a water-ethanol mixture. Thereafter, drying was carried out at 120 °C for 10 min. Subsequently, the capacitor bodies were impregnated with an aqueous poly(3,4-ethylenedioxy-thiophene)/polystyrene sulphonate solution (Clevios K V2 HV, Heraeus Epurio). Thereafter, drying was carried out at 120 °C for 10 min. Impregnation and drying of crosslinker agent solution and application and drying of Clevios K V2 HV were repeated one additional time.

### Process step F):

The capacitor bodies of process D) were then covered with a graphite layer and thereafter with a silver layer in order to obtain the finished capacitors in this way.

The ESR of the capacitor of process F) were measured initially and after the capacitors were exposed to 150°C for 164 hours. The ESR values for capacitors prepared with PEDOT-SNa and PEDOT-OSNa are given in Table 5.

**Table 5**

| | PEDOT-SNa | PEDOT-OSNa |
|---|---|---|
| ESR initial | 37 mΩ | 35 mΩ |
| ESR After 164h at 150°C | 94 mΩ | 55 mΩ |

### KEY TO REFERENCE NUMBERS

- 100: layered body
- 101: substrate

- 101a: porous electrode body
- 101b: electrode material
- 101c: dielectric layer

- 102: electrically conductive layer

- 102a: solid electrolyte layer

- 103: pores

## Claims

1. A monomer of the general formula (I) 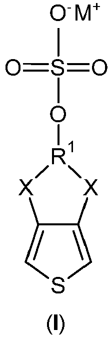 wherein
- X independently from each other represent O or S;
- R¹ represents a linear or branched trivalent organic group;
- M⁺ represents a cation, preferably a cation selected from the group consisting of H⁺, Li⁺, Na⁺, K⁺ and NH₄⁺, most preferably Na⁺.

2. The monomer according to claim 1, wherein the monomer has the general formula (I'): 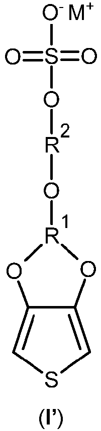 wherein
- R¹ represents a linear or branched trivalent alkyl group;
- **R²** represents a linear or branched divalent alkyl group, preferably a linear or branched divalent alkyl group is selected from the group consisting of -CH₂-CH₂-, -CH₂-CH₂-CH₂-, -CH(CH₃)-CH₂-, -CH₂-CH(CH₃)-, -CH₂-CH(CH₃)-CH₂-, -CH(CH₃)-CH₂-CH₂- and -CH₂-CH₂-CH(CH₃)-, wherein the first carbon atom in each alkylene group indicates the bond to the oxygen atom of the sulfate group.
- **M⁺** represents a cation, preferably a cation selected from the group consisting of H⁺, Li⁺, Na⁺, K⁺ and NH₄⁺, most preferably Na⁺.

3. The monomer according to claim 1 or 2, wherein the monomer has the general formula (I"a), the general formula (**I"b**) or wherein the monomer is a monomer mixture comprising monomers of the general formula (**I"a**) and monomers of the general formula (**I"b**): 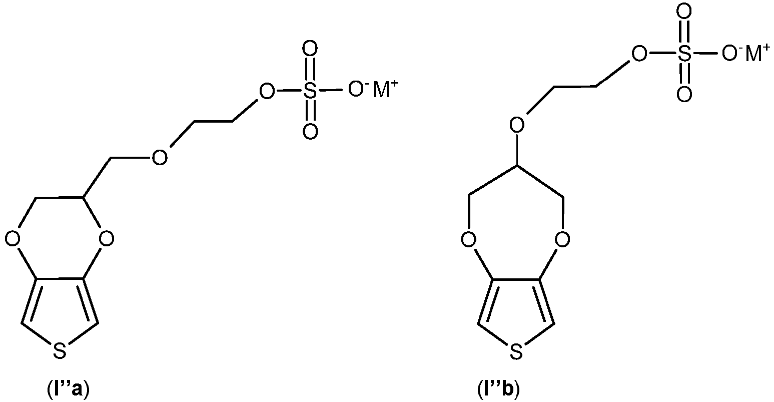 wherein **M⁺** represents a cation, preferably a cation selected from the group consisting of H⁺, Li⁺, Na⁺, K⁺ and NH₄⁺, most preferably Na⁺.

4. The monomer according to claim 3, wherein the monomer or the mixture of monomers comprises a compound of the general formula (**II**) 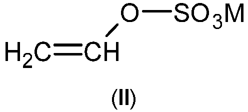
wherein **M⁺** represents a cation, preferably a cation selected from the group consisting of H⁺, Li⁺, Na⁺, K⁺ and NH₄⁺, most preferably Na⁺;
wherein the monomer or the mixture of monomers comprises the monomers having formula (**I"a**) or (**I"b**) and the compound of the general formula (**II**) in a molar ratio "monomer of formula (**I"a**) or (**I"b**)" : "compound of formula (**II**)" of at least 9:1.

5. An electrically conductive polymer comprising repeating units of the general formula (**III**) 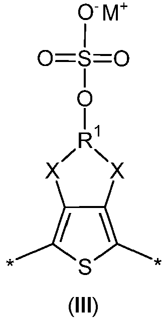 wherein symbol * indicates the bond to the neighboured repeating units and wherein
- **X** independently from each represent O or S;
- **R¹** represents a trivalent organic group;
- **M⁺** represents a cation, preferably a cation selected from the group consisting of H⁺, Li⁺, Na⁺, K⁺ and NH₄⁺, most preferably Na⁺.

6. The electrically conductive polymer according to claim 5, wherein the electrically conductive polymer comprises the repeating units of the general formula (III) in an amount of at least 75 mol-%, based on the total amount of repeating units in the electrically conductive polymer.

7. The electrically conductive polymer according to claim 5 or 6, wherein the electrically conductive polymer comprises repeating units of the general formula (**III"a**), of the general formula (**III"b**) or a combination of repeating units of the general formula (**III"a**) and of the general formula (**III"b**): 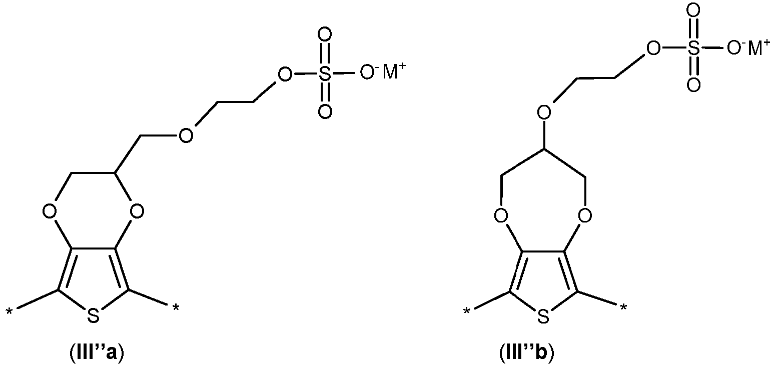 wherein* indicates the bond to the neighboured repeating units and wherein **M⁺** represents a cation, preferably a cation selected from the group consisting of H⁺, Li⁺, Na⁺, K⁺ and NH₄⁺, most preferably Na⁺.

8. The electrically conductive polymer according to claim 7, wherein the electrically conductive polymer comprises repeating units of the general formula (**III"a**) in an amount of at least 70 mol-% and repeating units of the general formula (**III"b**) in an amount of not more than 30 mol-%, in each case based on the total amount of repeating units in the electrically conductive polymer.

9. A liquid composition comprising
**i)** a dispersant;
**ii)** the electrically conductive polymer according to any of claims 5 to 8;

10. The liquid composition according to claim 9, wherein a conductive layer made from the liquid composition has a conductivity of at least 10 S/cm.

11. A process for preparing a layered body, comprising the process steps:
**A)** providing a substrate;
**B)** applying the liquid composition according to claim 9 or 11 onto a surface of the substrate;
**C)** at least partially removing the dispersant **i)** for the formation of a conductive layer that at least partially covers a surface of the substrate.

12. A layered body, comprising
**I)** a substrate;
**II)** a conductive layer that at least partially covers a surface of the substrate;
wherein the conductive layer comprises the electrically conductive polymer according to any of claims 5 to 8.

13. An electronic device, comprising the layered body according to claim 12.

14. The electronic device according to claim 13, wherein the electronic device is an electrolytic capacitor and wherein the conductive layer is a solid electrolyte layer or part of a solid electrolyte layer.

15. Use of the monomer according to any of claims 1 to 4, of an electrically conductive polymer according to any of claims 5 to 8 or of the liquid composition according to claim 9 or 10 for the formation of an electrically conductive layer.
